(19) Europäisches Patentamt
European Patent Office
Office européen des brevets

(11) EP 3 334 412 B1

(12) **EUROPEAN PATENT SPECIFICATION**

(45) Date of publication and mention of the grant of the patent:
**20.10.2021 Bulletin 2021/42**

(21) Application number: **16797943.4**

(22) Date of filing: **18.11.2016**

(51) Int Cl.:
***A61K 9/00*** *(2006.01)*
***A61K 47/32*** *(2006.01)*
***A61K 47/34*** *(2017.01)*
***A61K 47/36*** *(2006.01)*
***A61K 47/38*** *(2006.01)*
***A61K 31/00*** *(2006.01)*
***A61P 1/02*** *(2006.01)*
***A61P 15/02*** *(2006.01)*
***A61K 31/573*** *(2006.01)*

(86) International application number:
**PCT/EP2016/078151**

(87) International publication number:
**WO 2017/085264 (26.05.2017 Gazette 2017/21)**

(54) **A PHARMACEUTICAL COMPOSITION COMPRISING ELECTROHYDRODYNAMICALLY OBTAINED FIBRES, THE COMPOSITION HAVING IMPROVED RESIDENCE TIME ON THE APPLICATION SITE**

PHARMAZEUTISCHE ZUSAMMENSETZUNG MIT ELEKTROHYDRODYNAMISCH GEWONNENEN FASERN SOWIE VERBESSERTER VERWEILZEIT AN DER AUFTRAGUNGSSTELLE

COMPOSITION PHARMACEUTIQUE COMPRENANT DES FIBRES OBTENUES PAR UN PROCÉDÉ ÉLECTROHYDRODYNAMIQUE, PRÉSENTANT UN TEMPS DE SÉJOUR AMÉLIORÉ SUR LE SITE D'APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.11.2015 DK 201570745**

(43) Date of publication of application:
**20.06.2018 Bulletin 2018/25**

(73) Proprietor: **Afyx Therapeutics A/S**
**2300 København S (DK)**

(72) Inventors:
- **HANSEN, Jens**
  **2830 Virum (DK)**
- **ROMERO, Martin Eduardo Santocildes**
  **Sheffield S10 1QY (GB)**

(74) Representative: **Cooley (UK) LLP**
**22 Bishopsgate**
**London EC2N 4BQ (GB)**

(56) References cited:
**EP-A1- 2 810 645**
**WO-A1-01/27365**
**WO-A1-2014/066297**
**WO-A1-2015/189212**
**WO-A2-2004/014304**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the invention

The present invention relates to a pharmaceutical composition comprising drug-containing electrohydrodynamically obtained fibres, wherein the residence time - once applied to a humid surface or to a cavity containing a body fluid - is prolonged so as to ensure that the composition does not detach from the application site before it is desired. The invention also relates to the drug-containing electrohydrodynamically obtained fibres and a method for preparing the fibres and the pharmaceutical composition.

The fibres may be in the form of a layer and may be provided with one or more further layers, eg a backing layer that is insoluble in water or saliva and/or a layer that may influence the release of the drug substance from the final composition.

Moreover, the compositions are suitable for local application to internal wet surfaces such as vagina, vocal cord or the bowel eg for treatment of inflammatory bowel disease. Notably, the invention relates to compositions comprising electrohydrodynamically obtained fibres for application to the oral cavity to deliver a drug substance to the oral mucosa.

### Background of the invention

One of the major problems relating to treatment of diseases in the skin or mucosa is to deliver a correct amount of the drug substance to the diseased skin or mucosa. Compositions for use in the treatment of diseases in the skin or mucosa are very often in the form of a creme, an ointment or a gel, which is applied by the patient by spreading a variable amount of the composition on a diseased area of variable size, and the composition is spread on the area in a layer of variable thickness.

Accordingly, it is normally very difficult to obtain reliable results regarding eg relationship between dose and effect, inter- and intraindividual variations etc.

Transdermal systems like plasters are normally used for drug substances that must penetrate the skin, i.e. they are not intended for use in the treatment of diseases of the skin or mucosa, where the drug substances should act locally on or within the skin or mucosa. US 4,765,983 relates to an adhesive medical tape for use in stomatitis. The tape comprises a support layer consisting of an intestine soluble polymer and at least two medicament-containing layers consisting essentially of a water-soluble polymer containing a steroid.

The variability of dosage makes it very difficult to control treatment of a skin or mucosa disease and to make a correct decision regarding continuing or discontinuing treatment as it eg may be difficult to judge the benefit/risk profile for the treatment. If eg systemic side effects are observed then it is difficult to know whether the side-effects are due to over-dosing (the patient applies a too large dose by eg spreading the composition over a too large area, or the patient spreads the composition in a too thick layer) or whether the side-effects can only be avoided by termination of the treatment. Under-dosing may also be a problem in the topical therapy, especially when creams, ointments, lotions or other liquid or semi-liquid compositions are used. In general, 30% of patient undergoing topical treatment is subject to under-dosing.

In order to have a pharmaceutical composition approved by the regulatory health authorities, substantial documentation relating to the therapeutic effect, indication, side effects, toxic effects, dosages etc. is required. To this end it would be advantageous if effects relating to variability in dosages could be avoided or substantial reduced, i.e. if it is possible to ensure that the dose applied is controlled and not subject to the judgement of the patient applying the composition (eg area, thickness, frequency etc.). In this manner a more reliable benefit/risk profile could be obtained.

Moreover, in order to be effective the compositions for use in the treatment of diseases in the oral mucosa must stay on the diseased site for a specific period of time. Often compositions like eg films are not intended to be used in the treatment of diseases in the oral cavity, but are used to obtain a relatively fast absorption into the systemic circulation. Films normally disintegrate relatively fast, which either makes them unsuitable for use or they may be applied many times daily.

Another problem relates to administration to the oral cavity or other mucosa located in a humid environment such as vagina, ocular mucosa etc. Many diseases are located in the oral cavity and require local treatment (eg oral lichen planus). Such treatment is often by use of solutions, crèmes, pastes, or ointments, where it is administered by spreading the composition on the diseased area with a finger. In addition to the disadvantages mentioned above, saliva produced by the salivary glands together with movements of the tongue tends to remove the composition from the administration site and will reduce the effect of the treatment.

Thus, there is a need for developing compositions for treatment of a disease located on the skin or mucosa or as described in the Field of the invention, wherein the compositions are designed in such a manner that the composition not only has a suitable bioadhesiveness to the application site, but also has the ability to remain on the application site

for a desired period of time, i.e. the composition should not easily detach from the application site. Thus, the composition may stay on the application site for a prolonged period of time.

WO 2004/014304 A2 discloses electrospun amorphous pharmaceutical compositions. WO 01/27365 A1 discloses electrospun fibers and an apparatus for electrospinning a wound dressing. EP 2 810 645 A1 discloses a new drug delivery system. WO 2014/066297 A1 discloses nonwoven fiber materials. WO 2015/189212 A1 discloses compositions comprising electrohydrodynamically obtained fibres for administration of specific dosages of an active substance to skin or mucosa.

**Detailed description of the invention**

The present invention is a further development of the invention disclosed in PCT/EP2015/062842. The fibres and compositions described therein are based on the principle of employing a fibre-forming hydrophilic polymer together with a bioadhesive substance, where the fibre-forming hydrophilic polymer and the bioadhesive substance have very different solubility in the solvent that is used in the electrospinning process. The solubility of the bioadhesive substance in the solvent must be very low (slightly soluble) so that it is employed in the form of a suspension, whereby it is possible to make fibres having a relatively high content of bioadhesive substance in the fibres. The bioadhesive substance will be present in solid form and has not been subject to swelling or dissolution during manufacture, which has proven to be important in order to ensure a very strong bioadhesive effect.

However, during the development of fibres or compositions comprising fibres for application to wet surfaces in contact with body fluid (eg oral mucosa that is in contact with saliva) it is important to provide the fibres with a water-impermeable layer in order to ensure, that a drug substance, if present, is not released within the oral cavity, but only to the oral mucosa, i.e. it should only diffuse into the mucosa (eg diseased mucosa) and not anywhere else. However, it has been observed that although the bioadhesiveness may be strong, a certain detachment may take place, which is hypothesized to be due to hydration of the fibres caused by fluid from the mucosal surface upon which the fibres or fibre composition is placed. The swelling of the fibres may then have impact on the water-impermeable layer so that the edges loosen and, accordingly, eg in the oral cavity the tongue movements may increase the detachment time. If there is a high degree of hydration, a slippery, non-adhesive mucilage due to large amounts of saliva will lead to detachment of the fibre or fibre composition.

By incidence, WO 2015/189212 describes electrospun fibres made of PVP and Eudragit® RS100, wherein the weight ratio between Eudragit® and PVP employed is 0.5. No drug substance is added. In those jurisdictions where it is applicable, such electrospun fibres are not intended to be within the scope of the present invention and may thus be disclaimed.

The present inventors have found that the above-mentioned problem can be solved by

i) Increasing the amount of bioadhesive substance
ii) Increasing the thickness of the layer containing the bioadhesive substance
iii) Protecting the fibres or fibre composition against overhydration eg by

  a. Controlling the degree of hydration of the bioadhesive substance by controlling the influx of saliva into the fibres or fibre composition, and/or
  b. Protecting the fibres or fibre composition with a saliva resistant film, which is biodegradable.

The inventors have found that using two hydrophilic polymers with different solubilities in water solves the problem. By doing so it has been possible to protect the fibres or fibre composition from overhydration and to control the influx of body fluid (eg saliva) into the fibres or fibre composition.

Thus, in accordance with claim 1, the present invention provides electrospun fibres containing:

i) a first and a second hydrophilic fibre-forming polymer that is soluble in a hydrophilic solvent,
ii) a bioadhesive substance that is slightly soluble in said hydrophilic solvent,
iii) a drug substance,

wherein the first hydrophilic fibre-forming polymer has a solubility in water at 37°C that is at least 10 times greater than the solubility in water at 37°C of the second hydrophilic fibre-forming polymer, and wherein the bioadhesive substance is present in solid form.

Preferred features are defined in the dependent claims.

Preferably, a drug substance is present, but there may be situations (eg in aphtous ulcers or aphtous stomatitis or uncomplicated wounds eg in the oral cavity) where it is the protection of the disease area that is important and not necessary a treatment with a drug substance. All the details mentioned herein regarding the fibre-forming hydrophilic

polymer, the bioadhesive substance, concentrations thereof, ratios between the bioadhesive substance and the fibre-forming hydrophilic polymer and compositions comprising such fibres apply mutatis mutandis to the fibres or compositions with or without any content of a drug substance.

In PCT/EP2015/062842 is exemplified a fibre composition containing 33% Eudragit® RS100 and 67% PVP (Kollidone 90K). For jurisdictions where it may be relevant this composition is disclaimed from the present invention.

The permeability of water into the fibres or fibre composition may also be provided or enforced by cross-linking of said first hydrophilic fibre-forming polymer.

The European Pharmacopoeia uses the following terms to define the solubility of a substance in a particular solvent (Section 1.4, p. 7):

| Descriptive term | Approximate volume of solvent in mL per g of solute | | | |
|---|---|---|---|---|
| Very soluble | Less than | 1 | | |
| Freely soluble | From | 1 | To | 10 |
| Soluble | From | 10 | To | 20 |
| Sparingly soluble | From | 30 | To | 100 |
| Slightly soluble | From | 100 | To | 1000 |
| Very slightly soluble | From | 1000 | To | 10000 |
| Practically insoluble | More than | | | 10000 |

In some embodiments, the first and the second hydrophilic fibre-forming polymer has a solubility in said hydrophilic solvent of 3 g/100 ml or more at 25 °C or 10 g/100 ml or more at 25 °C. A suitable hydrophilic solvent is typically selected from ethanol, or ethanol-water mixtures. However, electrospinning may also be performed in other solvents such as eg acetone. If this is the case, the same solubility as mention above applies.

In some embodiments of those cases where ethanol-water mixtures are used in the electrospinning process, the water content should not exceed 20% v/v water. In general the concentration is less or 10% v/v water or less.

In preferred embodiments — and as seen from the examples, electrospun fibres are obtained using ethanol-water mixtures having a content of 5% v/v water or less such as 3% v/v water of less.

Regarding the difference in water solubility, the first hydrophilic polymer may have a solubility in water that is at least 50 times greater than the water solubility of said second hydrophilic polymer in water, both measured at 37°C.

Preferably, the first hydrophilic polymer has a solubility in water that is at least 100 times greater or at least 500 times greater than the water solubility of said second hydrophilic polymer in water, both measured at 37°C. The difference may also be so that the first hydrophilic polymer has a solubility in water that is at least 1000 times greater than the water solubility of said second hydrophilic polymer in water, both measured at 37°C.

As mentioned above, in order to achieve a strong and sufficient bioadhesion, in some embodiments the bioadhesive substance is at the most very slightly soluble in said solvent selected from ethanol, or ethanol-water mixtures at a temperature of 25 °C.

The solvent used in the electrospinning process may be C1-C3 alkanol such as methanol, ethanol, propanol or isopropanol, or acetone or mixtures thereof. The solvent or solvent mixture may also contain at the most 20% v/v of an aqueous medium such as water.

The bioadhesive substance may have a solubility of at the most 0.1% w/w in said solvent selected from ethanol, ethanol-water mixtures and at a temperature of 25 °C.

Preferably, the bioadhesive substance has a solubility of at the most 0.01% w/w in said solvent selected from ethanol, ethanol-water mixtures at a temperature of 25 °C.

The solvent or solvent mixture (in the following commonly denoted "solvent") used in the preparation of the fibres. Thus, to prepare the fibres the ingredients are contained in the solvent; the hydrophilic polymer is dissolved in the solvent and the bioadhesive substance is in undissolved form or at least 90% w/w of the bioadhesive substance is in undissolved form. The ingredients are dissolved/dispersed in the same type of solvent, but may be applied in the electrospinning process as one, two or three different mixtures.

The solubilities of the hydrophilic polymer and the bioadhesive substance in the solvent used are important in order to obtain the desired properties of the fibres of the invention. Thus, in some embodiments, the hydrophilic polymer must have a solubility in a first solvent of 3 g/100 ml or more at 25 °C or 10 g/100 ml or more at 25 °C, and the bioadhesive substance must have a solubility in said first solvent of 0.5 g/100 ml or less at 25 °C or 0.1g/100 ml or less at 25 °C.

Due to the difference in solubility the bioadhesive substance may be attached to the fibres as small particles. A small amount of the bioadhesive substance may be dissolved in the solvent and may therefore be an integral part of

the fibres, but in order to achieve maximal bioadhesive effect it is contemplated that the bioadhesive substance is attached to the fibres and that the fibrous structure essentially is due to electrospinning of the fibre-forming hydrophilic polymer. In some embodiments, at least 90% w/w of the bioadhesive substance is present in solid form.

Dependent on the properties of the drug substance it may be an integral part of the fibres or may be attached to or admixed with the fibres. Thus, if the drug substance is soluble in the solvent used and capable of forming fibres, then it may be an integral part of the fibres. If these conditions are not present the drug substance will be attached to the fibres of present in admixture with the fibres.

In the present context the term "integral part of the fibres" means that the substance together with the fibre-forming hydrophilic polymer form the fibrous structure of the fibres.

The hydrophilic polymer is the basic ingredient in the electrospun fibres and is the ingredient that has the ability to form a fibre material. In order to avoid any confusion with other ingredients present either in the electrospun fibres or in a composition thereof the term "fibre-forming hydrophilic polymer" is used in the following. The fibre-forming hydrophilic polymer is suitably a polymer that is soluble in or forms a gel in a $C_1$-$C_3$ alkanol such as methanol, ethanol, propanol or isopropanol, notably ethanol, propanol or isopropanol. The spinning process requires that the polymer, which is the main component of the fibres, is in dissolved form to allow a steady stream of the dissolved polymer to flow from a needle to a grounded collecting plate in a jet-like fashion during the spinning process.

Suitable fibre-forming hydrophilic polymers are polyvinylpyrrolidone (PVP), acrylates and acrylic copolymers (eg Eudragit®), and mixtures thereof. Other polymers like eg ethylcellulose (EC), hydroxypropylcellulose (HPC), or mixtures thereof may also be used. Ethylcellulose (EC), hydroxypropylcellulose (HPC), or mixtures thereof may especially be used in combination with polyvinylpyrrolidone (PVP) and/or acrylates includeing acrylic copolymers (eg Eudragit®). In the examples especially PVP and acrylic copolymers have been used.

In some embodiments, the first hydrophilic fibre-forming polymer is selected from polyvinylpyrrolidone (PVP), hydroxypropylcellulose (HPC) and mixtures thereof. The second hydrophilic fibre-forming polymer may be selected from acrylates, acrylic copolymers, ethylcellulose and mixtures thereof. In some embodiments, the first hydrophilic polymer is PVP, and the second hydrophilic polymer is an acrylic copolymer such as Eudragit®.

The concentration of the first hydrophilic fibre-forming polymer in the fibres is typically in a range of from 10 to 60% w/w, notably from 20 to 50% w/w or from 25 to 45% w/w.

The concentration of the second hydrophilic fibre-forming polymer in the fibres is typically in a range of from 10 to 60% w/w, notably from 20 to 50% w/w or from 25 to 45% w/w.

Increasing the concentration of the second hydrophilic fibre-forming polymer in the fibres leads to delayed or decreased hydration, and increasing the concentration of the first hydrophilic fibre-forming polymer leads to a faster and increased hydration.

In general, a weight ratio between the first and the second hydrophilic fibre-forming polymer in the fibres is from about 0.1 to about 6. In a preferred embodiment, the ratio is from about 1 to about 6.

The degree of hydration and the drug release must be balanced so that both a suitable drug release is obtained and the fibres or fibre composition does not detach from the application site until desired and an effective amount of drug substance, if present, has been delivered to the application site or to the desired site.

The total concentration of the first and the second fibre-forming polymer in the fibres is normally from about 40% to about 92% w/w notably from about 50% to about 85% w/w or from about 60% to 75% w/w.

Polyvinylpyrrolidone can be used in a grade having an approximate molecular weight of from 2,500 Da to 3,000,000 Da (eg Povidone with K-values of from 12 to 120). PVP can be purchased as Kollidon®:

| Kollidon® | Weight average molecular weight $M_w$ |
|---|---|
| 12PF | 2,000-3,000 |
| 17PF | 7,000-11,000 |
| 25 | 28,000-34,000 |
| 30 | 44,000-54,000 |
| 90F | 1,000,000-1,500,000 |

In the low MW-range suitable grades are contemplated to have a MW of from about 25,000 to about 120,000 Da, notably from about 70,000 to about 100,000 Da. In the examples herein Kolllidon® 90F has mainly be used and accordingly, a preferred PVP has a $M_w$ of from about 900,000 — about 3,000,000, notably from about 1,000,000 to about 1,500,000.

Ethylcellulose is sold under the trademark ETHOCEL™ (Dow Chemical Company) and is available in many different grades. Dow Chemical Company produces ethylcellulose in two ethoxyl types (denoted Standard and Medium).

Dependent on its ethoxyl content ethylcellulose may have different softening point and melting point temperatures. Ethylcellulose is also produced in a number of different viscosities. In the table below is given a listing of available ethylcelluloses.

ETHOCEL polymers

| Product viscosity designation | Viscosity range mPa*s | Ethoxyl content % Standard 48.0 — 49.5 | Ethoxyl content % Medium 45.0 — 46.5 |
|---|---|---|---|
| 4 | 3-5.5 | ETHOCEL Std. 4 | |
| 7 | 6-8 | ETHOCEL Std. 7 | |
| 10 | 9-11 | ETHOCEL Std. 10 | |
| 14 | 12.6-15.4 | ETHOCEL Std. 14 | |
| 20 | 18.22 | ETHOCEL Std. 20 | |
| 45 | 41.49 | ETHOCEL Std. 45 | |
| 50 | 45-55 | | ETHOCEL Med. 50 |
| 70 | 63-77 | | ETHOCEL Med. 70 |
| 100 | 90-110 | ETHOCEL Std. 100 | ETHOCEL Med. 100 |
| 200 | 180-220 | ETHOCEL Std. 200 | |
| 300 | 270-330 | ETHOCEL Std. 300 | |
| 350 | 250-385 | ETHOCEL Std. 4 | |

In plasticized form it has excellent thermoplasticity and is useful for compositions made by molding, extrusion or lamination. Ethylcellulose is also an excellent filmformer and is used in coating of eg tablets. The above-mentioned ethylcellulose qualities have an ethoxyl content of at least 45% and, accordingly they are soluble in ethanol and practically insoluble in water.

Acrylates and acrylic acid derivative include polymethacrylates, methacrylate copolymers, acrylic copolymers and methacrylate polymers. Preferred acrylates are those sold under the trademark EUDRAGIT®, which are soluble in ethanol, or acrylates/octaacrylamide copolymer (sold under the name DERMACRYL 79). These include EUDRAGIT®E 12,5 (amino methacrylate copolymer), EUDRAGIT® E100 (amino methacrylate copolymer; basic butylated methacrylate copolymer), EUDRAGIT®E PO ((amino methacrylate copolymer), EUDRAGIT®L 100-55, EUDRAGIT®L 100 (methacrylic acid — methyl methacrylate copolymer 1:1), EUDRAGIT®S 100 (methacrylic acid-methyl methacrylate copolymer 1:2), EUDRAGIT®RL 100, EUDRAGIT®RL 100 (ammonio methacrylate copolymer type A), EUDRAGIT®RL PO, EUDRAGIT®RS 100 (ammonio methacrylate copolymer type B), EUDRAGIT®RS PO. EUDRAGIT®E is a cationic polymer based on dimethylaminoethyl methacrylate and other neutral methacrylic acid ester: EUDRAGIT®L and S are methacrylic acid copolymers and are cationic copolymerization products of methacrylic acid and methyl methacrylate. EUDRAGIT®RL or RS is ammonio methacrylate copolymers synthesized from acrylic acid and methacrylic acid.

EUDRAGIT® E 100 is soluble up to pH 5.5 and E 12.5 is soluble above pH 5.

EUDRAGIT® L30 D-55, L-100-55 (methacrylic acid — ethyl acrylate copolymer 1:1), L 100, L 12,5, are normally used in enteric formulations, but may be used in order to delay release of the drug substance from fibres of the invention. EUDRAGIT® L30 D-55, and L-100-55 dissolve at a pH about 5.5 and the grades L 100 and L 12,5 dissolve at pH 6 or above.

As the pH in saliva normally is about 5-6 these polymers are of interest for fibres for oral use. If sustained or prolonged release is desired polymers being soluble at lower of higher pH may be more suitable for use.

EUDRAGIT® products are also available for sustained-release formulations and such grades may be of interest to incorporate in fibres of the invention either alone or together with another hydrophilic polymer. Relevant grades belong to the RL, RS, NE and NM series such as RL 100, RL PO, RL 30D, and RL 12,5, RS 100, RS PO, RS 30D, and RS 12,5, NE 30D and NE 40D, and NM 30D.

EUDRAGIT® RL 100 / EUDRAGIT® RS 100

Solid substances. EUDRAGIT® RL 100 (Type A) and EUDRAGIT® RS 100 (Type B) are described in the monographs quoted above. Eudragit® RS 100 is poly (ethyl acrylate-co-methyl methacrylate-co- rimethylammonioethyl methacrylate chloride) 1:2:0.1.

EUDRAGIT® RL PO / EUDRAGIT® RS PO

Solid substances obtained from EUDRAGIT® RL 100 or EUDRAGIT® RS 100. EUDRAGIT® RL PO (Type A) and EUDRAGIT® RS PO (Type B) are described in the monographs quoted above.

Chemical structure

EUDRAGIT® RL 100 / RL PO and EUDRAGIT® RS 100 / RS PO are copolymers of ethyl acrylate, methyl methacrylate and a low content of a methacrylic acid ester with quaternary ammonium groups (trimethylammonioethyl methacrylate chloride). The ammonium groups are present as salts and make the polymers permeable. The molar ratio of ethyl acrylate, methyl methacrylate and trimethylammonioethyl methacrylate is approx. 1:2:0.2 in EUDRAGIT® RL and approx. 1:2:0.1 in EUDRAGIT® RS.

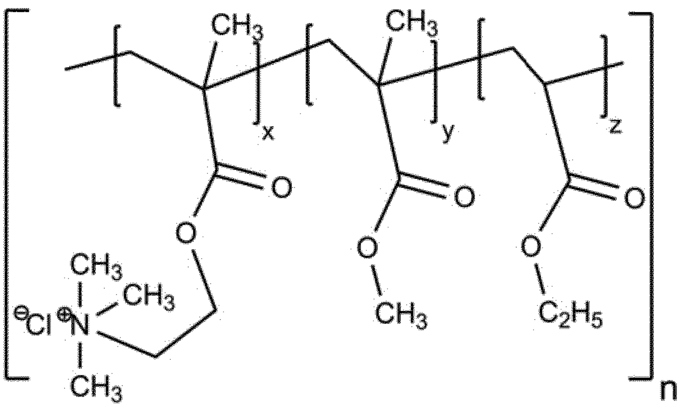

The monomers are randomly distributed along the copolymer chain. Based on SEC method the weight average molar mass (Mw) of EUDRAGIT® RL 100, EUDRAGIT® RL PO, EUDRAGIT® RS 100 and EUDRAGIT® RS PO is approximately 32,000 g/mol.

Characters

EUDRAGIT® RL 100 and EUDRAGIT® RS 100: colourless, clear to cloudy granules with a faint amine like odour.
EUDRAGIT® RL PO and EUDRAGIT® RS PO: white powder with a faint amine-like odour.

Solubility

1 g of the substances dissolves in 7 g aqueous methanol, ethanol and isopropyl alcohol (containing approx. 3 % water), as well as in acetone, ethyl acetate and methylene chloride to give clear to cloudy solutions.
The substances are practically insoluble in petroleum ether, 1 N sodium hydroxide and water.

Assay

Ph. Eur.:

EUDRAGIT® RL 100 and RL PO: 8.9 - 12.3 % ammonio methacrylate units on DS. EUDRAGIT® RS 100 and RS PO: 4.5 - 7.0 % ammonio methacrylate units on DS. The test is performed according to the Ph. Eur. monograph.

Viscosity / Apparent viscosity

$$1 - 15 \text{ mPa} * \text{s}$$

The viscosity of the Test solution is determined by means of a Brookfield viscometer (UL adapter / 30 rpm / 20 °C).
The test is performed according to Ph. Eur. 2.2.10 or USP <912> method II.

EUDRAGIT L 100-55

Solid substance. The product contains 0.7% Sodium Laurilsulfate Ph. Eur. / NF and 2.3% Polysorbate 80 Ph. Eur. / NF on solid substance.
EUDRAGIT® L 100-55 is described in the monographs quoted above.
EUDRAGIT® L 100-55 is the dry substance obtained from EUDRAGIT® L 30 D-55.

Chemical structure

EUDRAGIT® L 100-55 contains an anionic copolymer based on methacrylic acid and ethyl acrylate. The ratio of the free carboxyl groups to the ester groups is approx. 1:1.

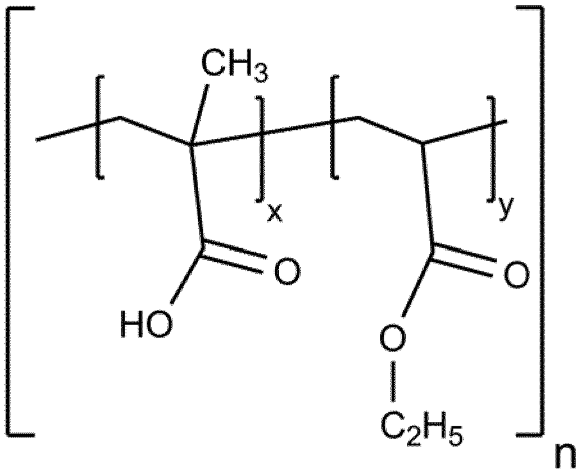

The monomers are randomly distributed along the copolymer chain. Based on SEC method the weight average molar mass (Mw) of EUDRAGIT® L 100-55 is approx. 320,000 g/mol.

Characters

White powder with a faint characteristic odour.

Solubility

1 g of EUDRAGIT® L 100-55 dissolves in 7 g methanol, ethanol, isopropyl alcohol and acetone, as well as in 1 N sodium hydroxide to give clear to cloudy solutions.
EUDRAGIT® L 100-55 is practically insoluble in ethyl acetate, methylene chloride, petroleum ether and water.

Viscosity / Apparent viscosity

$$100 - 200 \text{ mPa} * \text{s}$$

The viscosity of the Test solution is determined by means of a Brookfield viscometer (Spindle 1 / 30 rpm / 20 °C). The test is performed according to Ph. Eur. 2.2.10 or USP <912> method I.
Hydroxypropylcellulose is a non-ionic water-soluble cellulose ether. It combines organic solvent solubiltiy, thermoplasticity and surface activity and that thickening and stabilizing properties. The fibres are flexible and non-tacky at high humidity. Hydroxypropylcellulose is sold under the name KLUCEL™.
In the present context, the preferred fibre-forming hydrophilic polymers are selected from PVP, hydroxypropylcellulose (HPC), acrylates and acrylic acid derivatives, and mixtures thereof.
The concentration of the fibre-forming hydrophilic polymer(s) in the fibres according to the invention is normally

from about 40% to about 92% w/w notably from about 50 to about 85% w/w or from about 60% to 75% w/w.

Fibres of the invention also contain a bioadhesive substance. In order to ensure an easy manufacture of the fibres and to obtain the desired bioadhesive properties in situ after application to the mucosa, it is important that the bioadhesive in itself does not contribute significantly to the viscosity of a solution containing the fibre-forming hydrophilic polymer.

In the present context the term "bioadhesive" or "bioadhesion" indicates attachment to a specified biological location such as to the surface of the skin, a lip or a mucosal surface. A bioadhesive substance imparts bioadhesiveness to the drug-containing fibres of the invention or, in certain cases it may be included in a composition of the invention eg as a separate layer, which — after application — is the inner layer facing the skin or mucosa, i.e. the layer that is in contact with the skin or mucosa.

The bioadhesive substance for use in the present context can be selected from dextran, polyethylene oxides (PEOs), alginate, tragacanth, carrageenan, pectin, gelatin, guar, xanthan, gellan, methylcellulose, hydroxypropylmethylcellulose (HPMC), polyvinylalcohol (PVA), carboxymethylcellulose and alkali salts thereof, polymers of acrylic acids (PAA derivatives), chitosan, lectins, thiolated polymers, polyox WSRA, PAA-co-PEG (PEG is polyethylene glycol), and mixtures thereof.

In general it is expected that the adhesive effect of polymers increases with increasing molecular weight. Thus, in general adhesive polymers having relatively high molecular weight are preferred.

Polyethylene oxide can be used in grade having an approximate molecular weight of from 100,000 to 7,000,000. Preferred grades have an average molecular weight of from 100,000 to 4,000,000, or from about 700,000 to about 4,000,000. Polyethylene oxide is sold under the name POLYOX™ (Dow Chemical Company) with molecular weights ranging from 100,000 to 7,000,000 Da. As seen from the examples herein suitable polyethylene oxides have a molecular weight of 2,000,000 Da or more such as from 1,000,000 — 700,000 Da, or from 2,000,000 — 7,000,000 Da, or from 1,000,000 — 4,000,000 Da, or from 2,000,000-4000,000 Da, or about 2,000,000 Da.

Dextran can be used in grade having an approximate molecular weight of from 400,000 Da to about 2,000,000 Da. Preferred dextrans have a molecular weight of from about 500,000 to about 2,000,000 Da notably from about 700,000 to about 800,000 Da; from about 1,000,000 to about 2,000,000 Da; or about 2,000,000 Da.

Cellulose derivatives include hydroxypropylmethylcellulose, methylcellulose and carboxymethylcellulose.

Methylcellulose is sold under the name METHOCEL™ (Dow Chemical Company) and is available in a wide range of viscosity grades (from less than 3 to over 100,000 mPA*s).

HPMC is sold in various qualities depending on the viscosity. HPMC is sold under the names Metocel® and Klucel®. A suitable HPMC has an average molecular weight from about 80,000 to about 140,000.

Carboxymethylcellulose is available in a broad selection of grades. The viscosity ranges from 10 to 100,000 mPa*s. It is also available as its sodium salt with a broad range of substitution levels. Dow Chemical Company sells sodium carboxymethylcellulose under the name WALOCEL™.

Polyvinylalcohol can be used in grade having an approximately molecular weight of from 20,000 Da to 200,000 Da.

Preferred bioadhesive substances are polyethylene oxides, dextrans or combinations thereof.

The inclusion of a bioadhesive substance in the fibres according to the invention makes is possible to obtain a final formulation that is bioadhesive and can remain on the skin or mucosal surface for a prolonged period of time without falling off.

The amount of the bioadhesive substance in the fibres per surface area is important in order to ensure a suitable bioadhesion.

The concentration of the bioadhesive substance in the fibres may be from about 5% to about 60% w/w, notably from about 8% to about 50% or from about 10% to about 60%, from about 15% to about 60% or from about 15% to about 40% w/w, based on the sum of the total dry weight.

The present inventors have found that the weight ratio between the bioadhesive substance and the hydrophilic fibre-forming polymers in the fibres should preferably be in a range of from 0.1 to 10 such as from 0.2 to 10. It may depend on the particular hydrophilic polymer and the particular bioadhesive substance used, but the above mentioned range is normally applicable. The ratio will to a certain degree depend on the bioadhesive substance chosen so that the higher bioadhesive potential, the bioadhesive substance has, the lower ratio is required and vice versa. The numbers given are, however, regarded as general guidance. In the examples herein further examples are given. In particular, suitable results have been obtained when the weight ratio between the bioadhesive substance and the hydrophilic polymer is from 0.1 to 4 or from 0.1 to 2.

The fibres according to the invention also contain a drug substance. The drug substance is selected from drug substances, which are indicated for treatment of a disease of the skin, lip, or mucosa, or in the case, where the fibres are included in compositions for application on an internal surface as described here, the drug substance may be any drug substance that is indicated for the specific treatment. In the present context, the drug substance may be selected from drug substances, which are indicated for treatment of a disease in the oral or vaginal cavity such as a drug substance

that is indicated for local treatment of a disease in the oral cavity. Drug substances of particular interest are mentioned herein. The drug substance may be present in dissolved, undissolved or partly dissolved form dependent on the drug solubility in the hydrophilic polymer and bioadhesive substance used.

The fibres according to the invention may also contain one or more pharmaceutically acceptable excipients including those mentioned herein. Besides the excipients mentioned herein below, the fibres may contain a plasticizer. The plasticizer imparts a certain plasticity to the fibres, it may facilitate the manufacturing process and/or improve the flexibility and processability of the hydrophilic polymer(s). Examples of suitable plasticizers are citric acid esters like acetyl triethyl citrate, tributyl citrate or triethylcitrate, castor oil, diacetylated monoglycerides, dibutyl sebacate, diethyl phthalate, sorbitol, glycerol or glycerol derivatives like triacetin or tributyrin, a cellulose derivative like cellulose nitrate, glycols like polyethylene glycols notably polyethylene glycols with a molecular weight from about 100 to about 1500, polyethylene glycol monomethyl ether, propylene glycol, or mixtures thereof.

A plasticizer may affect the release rate of the drug substance. Accordingly, a plasticizer may also be regarded as a release rate modifier. Normally, a change in concentration of plasticizer will affect the release rate. Normally and if present the concentration of a plasticizer in the fibres is in a range of from 0 to about 10% w/w such as from about 0.5 to about 5% w/w.

The electrospun fibres may also contain a solubility improving agent in order to adjust or manipulated the release rate of the drug substance from the electrospun fibres. If present, the drug substance is dissolved in the solubility-improving agent and, optionally in one or more volatile solvents, notably a $C_1$-$C_3$ alkanol, before fed into the apparatus making the electrospun fibres. In this manner it is ensured that the solubility improving agent containing drug substance is located within the electrospun fibres. Suitable solubility improving agents include a polyoxyethylene fatty alkyl ester, an isopropyl ester of a straight or branched $C_8$-$C_{14}$ fatty acid, a propylene glycol mono- or diester of a $C_8$-$C_{14}$ alkanol or alkenol, a straight or branched $C_8$-$C_{24}$ alkanol or alkenol, a $C_6$-$C_{22}$ acylglyceride, N-alkylpyrrolidone or N-alkylpiperidone, and a mineral oil such a paraffin.

The polyoxyethylene fatty alkyl ester is suitably selected from the group consisting of polyoxyethylene-15-stearyl ether, polyoxyethylene-11-stearyl ether, polyoxyethylene-14-butyl ether, polyoxyethylene-10-cetyl ether, and polyoxyethylene-3-myristyl ether.

The isopropyl ester of a straight or branched $C_8$-$C_{14}$ fatty acid is isopropyl myristate, isopropyl palmitate, isopropyl isostearate, isopropyl linolate or isopropyl monooleate.

The propylene glycol mono- or diester of a $C_8$-$C_{14}$ alkanol or alkenol is propylene glycol monolaurate, propylene glycol monocaprylate or propylene glycol dipelargonate.

The straight or branched $C_8$-$C_{24}$ alkanol or alkenol may be capryl, lauryl, cetyl, stearyl, oleyl, linoyl or myristyl alcohol or 2-octyldodecanol.

The $C_6$-$C_{22}$ acylglyceride is a vegetable oil eg sesame oil, sunflower oil, palm kernel oil, corn oil, safflower oil, olive oil, avocado oil, jojoba oil, grape kernel oil, canola oil, wheat germ oil, almond oil, cottonseed oil, peanut oil, watnut oil or soybean oil, a highly purified vegetable oil eg medium chain triglycerides (caprylic/capric triglycerides), long chain triglycerides, castor oil, caprylic monoglyceride, caprylic/capric mono- and diglycerides or caprylic/capric mono-, di- or triglycerides.

N-alkylpyrrolidone is typically N-methylpyrrolidone and N-alkylpiperidone is typically N-methylpiperidone.

The solubility-improving agent may also be a fatty acid such as a medium, long or very long chain fatty acid including oleic acid and linoleic acid.

The concentration of the solubility improving agent in the electrospun fibres is — if present — in a range of from 0 to about 10% w/w such as from about 0.5 to about 5% w/w.

In some embodiments, the water content of the fibres is at the most about 5% w/w.

The thickness of the fibres (they are prepared as a layer) may be varied depending on the intended use. In order to ensure a suitable strength of the fibres, the thickness normally is in a range of from 10 micrometer to about 3 mm or to about 3-5 mm. The thickness is like the thickness of paper.

The thickness of the electrospun fibres (which appears as a sheet) is the same throughout the length and width of the sheet. In the present context the term "same" means that the difference in thickness over a length of 1 m and a width of 1 m is at the most 10%.

The bioadhesive substance and the drug substance are homogeneously distributed in the fiber material, which means that the concentration of the substances carried by the fibres (bioadhesive substance, drug substance and optionally the substances/additives mentioned herein) is the same per surface area, wherein the surface area is measured as length x width of a given part of the sheet of fibres

Each fibre contains an amount of the bioadhesive substance and the drug substance and, if other additives or ingredients have been employed, such a substance will also be part of the fibres.

The release of the drug substance from the fibres may be immediate release or modified release dependent on the specific drug substance and the intended use. The release rate may be adjusted eg to obtain a slower release by

i) use of fibre-forming hydrophilic polymer(s) with an increased average molecular weight,
ii) use of fibre-forming hydrophilic polymer(s) normally intended for use in sustained release compositions or enteric coated compositions,
iii) use of a mixture of fibre-forming hydrophilic polymers, wherein at least one of the polymers is insoluble in water or saliva
iv) increasing the concentration of bioadhesive substance to obtain a more compact fibre upon application to eg the oral cavity, where the bioadhesive substance may cause swelling,
v) increasing the compactness of the network structure in the fibres (alternatively cross-linking of the electrospun fibres,
vi) increasing the thickness,
vii) increasing the fibre diameter,
viii) changing manufacturing method (eg from simple needle nozzle to coaxial injection),
ix) applying a further layer eg of hydrophobic material on the fibre layer, which hydrophobic layer is intended to be applied closest to the oral mucosa and thus retarding the release of drug substance from the fibres.

A suitable hydrophobic material that can be used as a backing layer is poly(caprolactone).
Analogous, the release rate may be adjusted eg to obtain a faster release by

i) use of fibre-forming hydrophilic polymer(s) with an decreased average molecular weight,
ii) decrease the amount of bioadhesive substance to decrease the compactness of the fibres,
iii) increasing concentration of solubility-improving substance
iv) increase porosity of the fibres,
v) decreasing the thickness of the layer of fibres,
vi) decreasing the compactness of the network structure in the fibres,
vii) increasing concentration of solubility-improving substance,
viii) decreasing the diameter of the fibres,
ix) changing manufacturing method (eg from coaxial injection to simple needle nozzle).

The fibres according to the invention can be used in medicine, notably for the treatment of a disease located to the skin or mucosa.
In a specific aspect, the fibres according to the invention are for use in the treatment of diseases of the oral or vaginal cavity, notably for local treatment of the oral mucosa.
Such fibres are suitable for use in pharmaceutical compositions for application on the skin or mucosa for the treatment of diseases located to such areas. In the present context the term "mucosa" includes mucosa in the oral cavity, in the vagina, in the rectum, in the eye, in the ear as well as the lips. The fibres are also useful in compositions for application on internal surfaces such as e.g. organs (eg the liver, spleen, heart etc), tissues such as vocal cord, mucosa such as the gastrointestinal mucosa etc. Due to the nature of the electrospun fibres, the compositions of the invention can be provided for immediate release of the drug substance or for controlled release of the drug substance by varying the ingredients employed in the composition or in the electrospun fibres. The electrospun fibres typically become invisible after application or they appear as a plaster/patch, which makes it possible to apply the compositions on any part of the skin or mucosa such as in the face. It is also possible to apply eg cosmetics on the applied composition. This enables good patient compliance as the treatment does not leave any visible signs.
The invention also relates to pharmaceutical compositions comprising the electrospun fibres, to methods for obtaining the electrospun fibres and to use of the electrospun fibres and the pharmaceutical composition in medicine.
The use of electrospun fibres in medicine offers one or more of the following advantages:

i) It is possible to improve the therapeutic effect e.g. designing the compositions as a controlled release composition. In this manner the drug substance is released from the composition over a prolonged period of time and peak concentration of the drug substance at the applied site is avoided; such peak concentrations are very often responsible for un-desired effects such as irritation.

ii) The electrospun fibres are dry, i.e. there is no or only small amounts of water present in the composition. Moreover, the semi-solid compositions that normally are used for treatment of a disease in the skin or mucosa may contain excipients like vegetable oils, waxes, surfactants that may be subject to degradation. Degradation is normally faster if the composition contains a liquid solvent; thus, from a stability perspective, it is an advantage to develop compositions without or with only a minor amount of a solvent present. Accordingly, long shelf-lives are envisaged of the electrospun fibres and compositions of the invention.

iii) The method by which the fibres are electrospun enables fibres to be obtained with a content of more than one drug substance. The different drug substances may be added to the spinning process by injecting one composition containing all drug substances dissolved or dispersed in a solvent in the desired concentrations through one valve, or by using different valves to different drug substance (or a mixture of these two illustrative examples). Another possibility is to provide one layer of fibres containing one drug substance and then on top of this layer provide another layer of fibres containing a second drug substance. Thus, combination products with two or more drug substances can easily be obtained.

iv) The drug substance will be homogeneous distributed in the electrospun fibres; thus, a correct dosing is secured and can be expressed e.g. as amount drug substance per surface area.

v) The electrospun fibres and compositions are highly skin or mucosa friendly; the fibres become transparent or appear like a plaster/patch upon application and cosmetics can be applied on top of the fibres/compositions.

vi) The electrospun fibres/compositions are easy to apply. Normally, the composition contains three layers: a release-liner layer, a layer containing the electrospun fibres, and, optionally, a backing layer. The release liner layer serves as a protective layer for the drug-containing layer and is to be removed before application. The backing layer can be regarded as a coating that protects the composition from being removed from the application site (eg in the oral cavity by movements of the tongue or presence of saliva) or as an occlusive layer that drives the release of the active substance to the skin or mucosa.

vii) In contrast to compositions normally used to treat diseases of the skin or mucosa, the electrospun fibres and compositions of the invention do not smell.

viii) The electronspun fibres and compositions of the invention do not contain any or any substantial amount of alcohol or surfactants. The presence of such substances in topical or mucosal compositions often leads to irritation of the skin or mucosa.

ix) The electrospun fibres and compositions of the invention do not contain any preservatives.

However, it is contemplates that other methods such as other methods involving electrostatic forces may be used to obtain equal results. The overall term for such methods is electrohydrodynamic (EHD) methods and includes electrospinning, electrospraying, coaxial electrospinning, coaxial electrospraying, emulsion electrospinning, etc. Such methods are intended to be part of the present invention in relation to preparation of the fibres according to the invention.

*Pharmaceutical compositions*

As mentioned herein, the present invention also provides pharmaceutical compositions comprising the electrospun fibres described herein. In some embodiments, the concentration of the electrospun fibres in the composition is from 70 to 100% w/w.

The pharmaceutical compositions are intended for use on the skin or on a mucosal surface, notably a mucosal surface of the oral cavity. A composition of the invention is typically in the form of a sheet containing one of more layers, where at least one layer contains the electrospun fibres and wherein the electrospun fibres contain the drug substance. The composition may be provided in the form of a sheet. It may be have a round, elongated or polygonal shape. The composition or the invention is a dosage form, which could be denoted sheet, layered composition, membrane, or patch.

In a simple form the composition only contains one layer, namely the layer of drug-containing electrospun fibres. Such a composition is suitable for use on the skin. After application the composition stays on the application site due to its bioadhesive character and it becomes transparent.

The composition may also contain more than one layer such as two or three or more layers. If the composition for example contains two layers, each layer may be a layer of drug-containing electrospun fibres, where the drug substance in the two layers may be the same or different. The two layers may also have different composition with respect to nature and content of fibre-forming hydrophilic polymers and/or bioadhesive substances in order to facilitate a different release pattern of the drug substance from the two different layers. Another example is that the composition contains one or more layer(s) of drug-containing electrospun fibres and another therapeutically inert layer, which functions as a backing layer to protect the drug-containing layer(s) from moisture or saliva or to function as an occlusive layer, which may drive the penetration of the drug substance into the skin or mucosa. In case, where such a composition is applied to the oral mucosa, a backing layer protects the drug-layer from being washed away from the application site, which would result in swallowing of the composition, whereby the desired local therapeutic effect is reduced or eliminated.

Alternatively, the composition may contain a layer, wherein a specific area is made up of one type of electrospun fibres and another specific area is made up of another type of electrospun fibres.

In some case it may be desired to have one or more layers of electrospun fibres without any content of a drug substance between the layer(s) of drug-containing electrospun fibres and/or a backing layer. Such layers of electrospun fibres may have the same composition as the layer of drug-containing electrospun fibres, but without any content of drug substance, or the composition may be different eg containing a fibre-forming hydrophobic polymer or a mixture of a hydrophobic and fibre-forming hydrophilic polymer. It is envisaged that such a layer may be used to adjust the release of the drug substance from the composition. Thus, such a composition is of particular interest in the case where a controlled release composition is desired. In this manner it is contemplated that an improved ratio between side effects and clinical effect can be obtained, i.e. it is possible to reduce the unwanted effects and at the same time achieve a therapeutically effective response.

The backing layer is typically either co-spun with the drug-containing layer or it is provided as a coating layer on top of a drug-containing layer. Typically, the backing layer is water-impermeable to enable an occlusive effect and/or a protective effect against eg saliva. Suitable materials for backing layer include polyethylene-co-vinyl acetate, ethylcellulose, poly(caprolactone), carbothane or polysoftane. Moreover, materials such as actylates/octylacrylamide copolymer (sold under the name DERMACRL® 79), amino methacrylate copolymer (EUDRAGIT®), dimethylaminoethyl methacrylate, methacrylate, methyl methacrylate (e.g. EUDRAGIT ®E 100) and other acrylates may be used or added. Plasticizers like those mentioned herein before (e.g. tributyl citrate) can also be added.

The backing layer, if present, normally has a thickness in the same order of magnitude as the composition. The backing layer, if present, normally make up about 30-60% w/w of the composition.

The composition may be subjected to heat treatment in order to melt the substance contained in the backing layer. The effect thereof is to obtain a closer structure of the backing layer in order to avoid penetration of water (or saliva or another relevant body fluid) into the composition and thereby avoiding the risk of releasing the drug substance too fast or avoiding the risk of unwanted separation of the backing layer from the drug-containing layer. The temperature employed should be a balance between obtaining melting of the substance in the backing layer and avoiding unwanted degradation of the drug substance. Poly(caprolactone) melts at about 65 °C.

A composition of the invention may be provided with a release liner layer. This layer is not part of the composition and is an inert layer, which must be removed before application on the skin or mucosa. The release liner layer only serves a practical purpose as it is difficult to handle and to pack a sheet of electrospun fibres without protecting the composition from the environment. Thus, if the composition only contains one layer, i.e. the layer of drug-containing electrospun fibres, it may be provided with a release liner layer both on the two outermost surfaces of the layer

The electronspun fibres and/or the compositions containing the fibres may also contain one or more pharmaceutically acceptable excipients, some of which have already been disclosed herein and they can also be added to a composition of the invention so that they are part of the composition, but not contained inside the electrospun fibres.

Such excipients (which also may be used in the preparation of the electrospun fibres) include taste-masking agents such as aromas or sweetening agents; pH adjusting agents such as buffer substances like citrates, acetate, or phosphate; release modifiers; pore-forming agent, stabilizing agents; anti-oxidants; pigments; skin conditioning agents including urea, glycerol etc, anti-irritative agents such as glycerol, menthol, eucalyptol or nicotinamide; anti-nucleating agents such as glycerol,; penetration enhancers such as azone, N-methylpyrrolidone, propylene glycol etc.

The release of the drug substance from the composition may be immediate or modified dependent on the particular drug substance employed and the intended use. The release rate may be adjusted as described herein before under the heading "Electrospun fibres", and/or it may be adjusted by use of specific pharmaceutically acceptable excipient.

A faster release may be obtained by use of penetration enhancer and/or by inclusion of a plasticizer.

A composition of the invention suitable for use on the skin or mucosa is typically composed of

i) from about 75-100% w/w of the drug-containing electrospun fibres
ii) from about 0-25% w/w of one or more pharmaceutically acceptable excipients (as described herein),

A composition of the invention suitable for use on the skin or mucosa is typically composed of

i) from about 50-70% w/w of the drug-containing electrospun fibres
ii) from about 0-10% w/w of one or more pharmaceutically acceptable excipients (as described herein), and
iii) from about 30 to 50 % w/w of a backing layer.

*Method for preparing fibres according to the invention*

The present invention also provides methods for preparing electrospun fibres.

A first method comprising

i) dissolving the first and the second hydrophilic polymer in a solvent selected from ethanol or ethanol-water mixtures,
ii) suspending the bioadhesive substance in the resulting solution from step i)
iii) if relevant, adding a drug substance to the resulting dispersion from step ii)
iv) electrospinning the resulting mixture from step ii) or iii),

wherein said first and second hydrophilic polymer is soluble in said solvent, and said bioadhesive substance is slightly soluble or less in said solvent,
to obtain electrospun fibres, wherein at least 90% w/w of the bioadhesive substance is present in solid form.

An alternative method for preparing electrospun fibres according to the invention comprises the steps of

i) dissolving the first and second hydrophilic polymer in a solvent selected from ethanol, water or mixtures thereof to obtain a first solution,
ii) if relevant, dissolving or suspending a drug substance in said first solution to obtain a first mixture,
iii) suspending the bioadhesive substance in the solvent to obtain a dispersion,
iv) dual-electrospinning the mixture from step i) or if relevant step ii) and the dispersion from step iii),

wherein the first and second hydrophilic polymer is soluble in first solvent, and said bioadhesive substance is slightly soluble in said solvent,
to obtain electrospun fibres, wherein at least 90% w/w of the bioadhesive substance is present in solid form.

In the above-mentioned methods, the first and second hydrophilic fibre-forming polymers may be dissolved in the same step i). Alternatively, they may be dissolved separately and either admixed before spinning or electrospun via different nozzles.

As mentioned hereinbefore a suitable solvent is one or more volatile solvents, notably a ethanol or ethanol-water mixtures. Water may be present up to about 20% v/v notably from about 3 to about 10% v/v. In those cases where the fibre-forming hydrophilic polymer and the bioadhesive substance are spun by dual-electrospinning, i.e. from two separate syringes, water may be used in concentrations up to about 60% v/v, notably up to about 50% v/v or up to about 40% v/v. In such cases the solvent for the fibre-forming hydrophilic polymer and the bioadhesive substance is not the same as the solvent used for the bioadhesive substance must be a solvent in which the bioadhesive substance is only slightly soluble or less than slightly soluble. A suitable solvent in which the bioadhesive substance is not soluble is notably ethanol or ethanol-water mixtures with a water content up to about 20% v/v, notable from about 3 to about 10% v/v.

The concentration of the fibre-forming hydrophilic polymer in the first solvent is typically in a range of from about 2 to about 40% w/w, notably from about 3 to about 30% w/w.

The concentration of the bioadhesive substance in the first solvent or in the second dispersion is typically from about 1 to about 20% w/w notably from about 1 to about 15% w/w.

The methods mentioned above may include a final step of coating an outer surface of the fibres with a hydrophobic polymer.

The coating may be in form of spraying, film casting, electrospinning etc.

After coating, the coated fibres may be subject to heating to melt or soften the hydrophobic polymer in order to obtain a more closed structure of the hydrophobic polymer.

The present invention also relates to a kit as described in the claims.

*Use in medicine*

The drug-containing electrospun fibres and the compositions containing the drug-containing electrospun fibres are suitable for use in medicine.

As mentioned above, the drug-containing electrospun fibres and compositions are primarily intended for local administration to a diseased site on the skin or on a mucosa. However, it is envisaged that a person skilled in the art and based on the present disclosure will be able to utilize the concept of present invention to obtain compositions that enable delivery to the systemic circulation after administration to the skin or mucosa or compositions that enable delivery of the drug substance to a body cavity such as the oral cavity. However, the object of the present invention is to provide electrospun fibres and compositions that stay on the diseased tissue to obtain a local effect.

Drug substances suitable for use in connection with the present invention may be drug substances that are small molecules or it may be peptides, proteins, biologics including mono- or polyclonal antibodies.

*Skin diseases*

Examples of skin diseases are actinic keratosis, skin cancers (basal cell carcinoma, Bowen's disease, squamous cell carcinoma, and malignant melanomas), genital warts, acne, dermatitis, psoriasis, rosacea, ichtyoisis, eczema, atopic

dermatitis, puritus, pustolis palmophantatis, pain, infections, viral diseases such as herpes.

Today some of these skin diseases (actinic keratosis, skin cancers (basal cell carcinoma, Bowen's disease, squamous cell carcinoma, and malignant melanomas), genital warts) may be treated with imiquimod, which is a prescription medication that acts as an immune response modifier. It has also been suggested to be used in the treatment of vulvar intraepithelial neoplasia, vaginal intraepithelial neoplasia, and common warts. However, there are several adverse effects of the treatment such as blisters, bloody dry eschar, pain and general discomfort. Moreover, many of the patients cannot tolerate the treatment.

Another treatment of actinic keratosis is ingenol.

A gel containing ingenol mebutate is on the market today in two different strengths for use on either the face and scalp (0.015%) or the trunk and extremities (0.05%), respectively. Clinical studies have shown has ingenol mebutate gel applied topically for 2 to 3 days is effective for field treatment of actinic keratosis.

Ingenol mebutate is sold under the name Picato®. The substance is an ester of the diterpene ingenol and angelic acid. Ingenol mebutate is practically not absorbed through the skin.

However, application of the gel very often leads to irritations of the application site. This includes redness, scaling, crusting, pain, and sometimes infection. Other side-effects include eye irritation such as periorbital edema, headache and nasophyryngitis.

Due to the common side-effect of irritation of the application site there is a need for developing a composition containing ingenol mebutate or another ingenol derivative which upon application to the skin is less irritative than the known composition. Moreover, a composition of the invention containing eg ingenol mebutate or imiquimod and being in the form of a sheet with a well-defined area (i.e. it contains the desired dose of the drug substance) may have improved long term and less recurrence due to correct dosing at every application.

A composition suitable for use typically comprises electrospun fibres, wherein the fibres are based on PVP and additionally contains a fibre-forming agent, a plasticizer, an anti-irritative agent and the drug substance. When imiquimod is the drug substance it may be present in the fibres as a dispersion or a solution, where e.g. oleic acid is used as a solvent. A typical example of a hydrophilic fibre-forming agent is an acrylate (eg as described herein) or PVP. The plasticizer may be tributyl citrate and the anti-irritative agent may be glycerol.

Other drug substances used in the treatment of skin diseases and suitable for use in accordance with the present invention are vitamin D derivatives or analogues, corticosteroids, phosphodiesterase 4 inhibitors , ingenol derivatives, retinol such as adaplene, JAK inhibitors, NK-1 receptor antagonists, calcineurin inhibitors such as tacrolimus or picrolimus, keratolytic agents such as salicylic acid or lactic acid, antibiotics such as fucidic acid, bactoban, or clindamycin, non-steroidal antiinflammatory agents such as diclofenac, naproxene, ibuprofen, ketoprofen, anti-neoplastic agents such as 5-fluoracil, local anesthetics such as lidocain, xylocaine, prilocain etc.

*Diseases of mucosa, notably the lips and oral cavity*

Diseases of the oral cavity that can be treated with the electrospun fibres or compositions of the invention include: Inflammatory conditions such as oral lichen planus and mouth ulcers. Such conditions are normally treated with corticosteroids. The corticosteroid may be selected from the group consisting of amcinonide, betamethasone, budenoside, clobetasol, clobetasone, cortisone, desonide, desoxycortisone, desoximethasone, dexamethasone, diflucortolon, diflorasone, flucortisone, flumethasone, flunisolide, fluocinonide, fluocinolon, fluorometholone, fluprednisolone, flurandrenolide, fluticasone, halcinonide, halobetasol, hydrocortisone, meprednisone, methylprednisone, mometasone, paramethasone, prednicarbate, prednisone, prednisolone and triamcinolone or a pharmaceutically acceptable ester or acetonide thereof. The corticosteroid may preferably be selected from betamethasone, budenoside, clobetasol, clobetasone, desoximethasone, diflucortolon, diflorasone, fluocinonide, fluocinolon, halcinonide, halobetasol, hydrocortisone, mometasone and triamcinolone or a pharmaceutically acceptable ester thereof. The corticosteroid ester may for instance be betamethasone acetate, betamethasone dipropionate, betamethasone valerate, elobetasol propionate, dexamethasone acetate, flumethasone pivalate, fluticasone propionate, hydrocortisone acetate, hydrocortisone butyrate or mometasone furoate. The acetonide may be selected from fluocinolone acetonide or triamcinolone acetonide. The corticosteroid is preferably betamethasone dipropionate or betamethasone valerate.

Pain conditions (treatment with analgesics such as NSAIDs - ibuprofen, ketoprofen, diclofenc etc.).

Fungal diseases (treatment with metronidazole, ketoconazole etc.).

Viral diseases such as herpes simplex (treatment with acyclovir).

Various dysplasia conditions (treatment with 5-fluoruracil, diclofenac, retinoids, ingenol mebutate) .

In the following is given a more specific description of the clinical applications for treatment of oral diseases.

*Use as simple wound dressings (with or without incorporated drugs)*

(a) Oral Ulceration

The oral mucosa is frequently traumatised during mastication and as the result of normal, chemical and physical injury. This usually leads to ulceration of the oral mucosa. The ulcerated area is painful, very sensitive to touch, hot foods and drinks, alcohol and strong or spicy flavours. This can be very uncomfortable and make eating, drinking and speech difficult. In addition, around 25% of the population experience recurrent episodes of oral ulceration (known as aphthous ulceration) at some point during their lives. They experience one or several mouth ulcers at a time that develop spontaneously, last a few days to a few weeks and then heal by themselves. These crops of ulcers recur frequently.

As with a wound to the skin, there is a natural instinct to cover such wounds in the mouth. Unfortunately, the equivalent of a Band-Aid does not yet exist for the mouth. Thin and flexible electrospun compositions that adhere to the oral mucosa and provide a degree of protection to the wound from the spicy foods, strong flavours etc that pass through the mouth as well as providing a degree of protection from bacterial contamination and physical trauma would speed wound healing and provide relief from the discomfort associated with oral ulcers. Ideally, these compositions should resorb slowly over a few days so that removal is not necessary. Healing of protected wounds in the mouth is generally very fast.

In some cases it is relevant to use electrospun fibres without any content of drug substance and, thus, the present invention also relates to such electrospun fibres (as described in detail herein, but without any content of drug substance), to compositions comprising the electrospun fibres and to the use of the fibres and compositions in medicine, i.e. not only to the above-mentioned appliance.

(b) Wound dressing following surgery

Surgical procedures in the mouth, particularly extractions, are more common than any other form of surgical procedure. Currently, following a simple tooth extraction, the open socket is left unprotected to form a blood clot and heal by itself. Fortunately, healing in the mouth is very effective. None-the-less, post extraction haemorrhage is common — often due the blood clot being dislodged, infection of the tooth socket — leading to delay in wound healing or the very painful condition of 'dry socket', is also common. Patients also dislike the sensation of an open socket in the mouth and the associated taste of blood. Covering the extraction socket with an adhesive electrospun composition eg in the form of a dressing, would help to keep the forming blood clot in place and so reduce post-extraction haemorrhage and improve wound healing. It would also reduce infection and the entry of food debris into the socket again facilitating wound healing and reducing wound infection. As well as physically covering the open socket providing comfort and reassurance to the patient. Such compositions would require good adhesion, need to have good strength, low permeability and ideally to stay in place for the first 24 hours while the blood clot stabilises.

As well as extractions many other surgical procedures are performed in the mouth, including, biopsies, gingival surgery, surgical extractions, implant surgery, orthodontic surgery etc. All leave open wounds or areas of suturing where suitable wound dressings would help reduce wound infection and secondary haemorrhage as well as providing physical protection and comfort for the patient.

(c) Active wound dressings

Although physical protection alone would have considerable benefit, the incorporation of drugs and other active agents in some wound dressings would have particular value in specific situations:

(i) Antiseptics. As secondary infection is a common issue with oral wounds, the incorporation and slow release of a well-tolerated antiseptic agent such as chlorhexidine gluconate or cetylpyridinium chloride (used in antiseptic mouthwashes) could be of value in situation where secondary infection is a particular issue.

(ii) Analgesics. Most oral ulcers are associated with pain and inflammation so the incorporation and slow release of a well-established topical analgesic/anti-inflammatory agent such as benzydamine hydrochloride could provide pain relief and a soothing effect as well as physical coverage.

(iii) Haemostatic. Post extraction haemorrhage is a common problem of concern to dentists and patients. Where haemorrhage is difficult to control with simple measures such as direct pressure. In such situations dentists and oral surgeons often use tranexamic acid — which inhibits fibrinolysis. However, because it comes in a tablet form it is difficult to apply locally to the tooth socket and so its main effect is systemic. Release of tranexamic acid from a socket covering composition eg in the form of a sheet or patch would physically prevent/reduce haemorrhage as well as preventing fibrinolysis locally in the socket whilst minimising the likelihood of any systemic effect. The composition would amplify the local effect of transexamic acid by preventing its loss from the socket.

*Actinic Keratosis and Oral Leukoplakia*

Actinic Keratosis (Solar Keratosis) is UV light induced premalignant lesion of the lip that has a significant risk of developing into a lip cancer. Such lesions are often surgically excised or treated with cryotherapy but recently the application of Imiquimod (Aldara), diclofenac (Solaraze) and Fluorouracil (Efudix) creams has been shown to be of benefit in treating some cases of actinic keratosis. However, better methods of retaining, localising and slowly releasing the active agents are needed than is achieved with the creams. Thus there is an interest in incorporating these drugs into electrospun compositions that can cover the area of actinic keratosis and slowly release the active agent for improved treatment.

Oral leukoplakia is a potentially malignant lesion of the oral mucosa that has a significant risk of converting to oral cancer. Oral leukoplakias are more common than actinic keratosis and occur more frequently in smokers. Their potential for malignant change is usually assessed by taking a biopsy of the lesion. A histopathologist then grades the degree of dysplasia in the lesion. Those lesions exhibiting moderate or severe dysplasia are considered at high risk of progressing to cancer. Current treatment of oral dysplastic lesion involves risk reduction e.g. stopping smoking and if the lesion is considered at high risk then surgical excision. Since oral leukoplakias can be extensive and it is difficult to access regions of the mouth, surgical treatment can be difficult and may be mutilating and unpleasant for the patient often leaving residual morbidity. Moreover, surgical removal may not reduce the risk of an oral cancer developing. Attempts have been made to use Imiquimod (Aldara), diclofenac (Solaraze) and Fluorouracil (Efudix) creams to treat oral leukoplakias. However, the presence of saliva make application and retention of the creams difficult and the large size of many oral leukoplakias and the risk of swallowing the drug compound the difficulties as well as significantly increasing the risk of systemic side effects from the drugs. Localised, slow release delivery via a bioadhesive electrospun composition of the invention, directly to the lesion would solve many of these problems particularly if the composition has an impermeable backing to ensure unidirectional delivery of the drug into the lesion and not into the oral cavity where it could be swallowed.

*'Cold Sores'*

Between 40% and 70% of the population (depending on geographic area and socioeconomic group) are latently infected with the herpes simplex type 1 virus. Of these, 20-40% experience periods of reactivation of the virus which most commonly presents as cold sores. In many individuals, cold sores are a common and reoccurring problem. Most often they are present as a crop of vesicles (small blisters) at the junction of the lip and the surrounding skin. These then rupture to form small ulcers that exude fluid and then crust over before healing spontaneously over 7 to 10 days. They cause considerable, pain, discomfort and embarrassment. If treated with topical antiviral agents such as acyclovir (Zovirax) or Penciclovir (Vectavir) cream at the earliest stages of lesion development the duration and severity of episodes can be reduced. However, the cream needs to be applied very frequently (respectively 5 $\times$ daily and 2 hourly) to be effective and does nothing to provide coverage to prevent secondary infection or hide the appearance of the lesions. Small electrospun skin adhesive compositions delivering slow release acyclovir or a similar antiviral agent, would be more effect and would also provide coverage to hide the lesions and prevent secondary infection.

Less commonly, cold sores form small, localised crops of blisters and ulcers on the lining mucosa of the mouth, most often the roof of the mouth. Again these are very painful but much more difficult to apply an anti-viral cream to. With appropriate formulation in accordance with the present invention, a composition of the invention can used to treat cold sores on the lips could also be used to treat lesions within the mouth

*Treatment of Immunologically Mediated Oral Mucosal Disease*

There are several common immunological mediated oral mucosal diseases that result in extensive areas of oral mucosal erosion (thinning) and ulceration. Wherever there is thinning or ulceration of the oral mucosa it is painful, very sensitive to touch, hot foods and drinks, alcohol and strong or spicy flavours. This can be extremely uncomfortable and make eating, drinking and speech difficult. As previously discussed, simple coverage of such areas can provide considerable relief. However, these conditions are either recurrent — such as recurrent aphthous stomatitis, or chronic. Therefore, suppression of the underlying disease process is essential if lesion improvement is to occur with longterm improvements for the patient.

Many of these conditions are susceptible to immunomodulatory drugs such as steroids, cyclosporine and mycophenelate mofetil. Generally steroids are the first line of therapy but there are virtually none formulated for topical drug delivery to lesions in the mouth, particularly as creams and ointments will not adhere to the oral mucosa and therefore easily swallowed and have virtually no duration of action where needed. As a result, steroid tablets (prednisolone and betamethasone valerate) are dissolved in water to make mouthwashes or steroid inhalers are directed at affected areas of oral mucosa. However, the contact time of drugs delivered in this way to oral lesions is extremely short and so

high doses, high potency steroids and frequent application is required to compensate for this. In turn this increases the risk of both oral and systemic side effects. Indeed, for more severe and difficult to treat lesions it is often necessary to resort to the use of systemic steroids to treat a localised disease. Even then, many patients ara resistant to treatment and it is often necessary to turn to more potent or steroid sparing alternative immunomodulatory drugs such as azathioprine, cyclosporine and mycophenelate mofetil. Again there are no topical oral preparations of these drugs so they frequently have to be used systemically.

Because the oral lesions are superficial and easily accessible modern biological agents such as antibodies and kinase inhibitors that often have to be given parenterally (by injection) could be applied directly to the lesion and have an effect if they were available in a suitable delivery system.

Thus electrospun bioadhesive compositions in accordance with the present invention providing uni-directional drug delivery into the lesion would provide much needed and effective treatment of a wide range of oral mucosal diseases. In terms of the most widely applicable drug to incorporate into a sheet a steroid preparation would be the best starting place. Hydrocortisone has the benefit of no significant absorption from the gut. In general it is of too low potency to be effective for oral mucosal diseases but with longer retention times and slow release it may well prove effective when delivered from a uni-directional patch. Stronger steroid preparations however, are widely used including trimacinolone acetonide - that has a proven track record as a topically delivered medium potency steroid (used to be available as triamcinolone in OROBASE® for topical delivery to oral lesions — but is no longer available). Otherwise betamethasone or fluocinolone have increasing potency and are widely used for treating oral mucosal disease currently. While steroids and other immunomodulatory drugs suppress the underlying disease process they are not effective at providing immediate symptomatic pain relief. Therefore a combined steroid and topical analgesic/antiinflammatory (benzidamine hydrochloride) drug delivery membrane could be of particular value.

Specific oral mucosal diseases suitable to being treated with a composition in accordance with the present invention include:

(i) Recurrent aphtous stomatitis — as previously described

(ii) Oral lichen planus (OLP) — This condition affects 1.5 — 2% of the population. Unlike the skin form of lichen planus, Oral lichen planus once established lasts for many years, causes far more painful lesions and is much more resistant to treatment. Patients get widespread erosions and ulceration that affects mainly the buccal mucosa (inside the cheeks), the sides of the tongue and the gums that are often painful and extremely sensitive to foods etc.

(iii) Pemphigoid — this is a group of blistering conditions that can affect the skin and mucous membranes. It is caused by auto-antibodies damaging the junction between the epithelium and the underlying connective tissue so that the epithelium splits from the underlying tissue. The oral mucosa is invariably affected producing large blisters that break down to form extensive areas of oral ulceration. The gums are widely affected but ulcers can also develop on the roof of the mouth tongue and inside the cheeks. It is somewhat less common the OLP.

(iv) Pemphigus — this is another blistering condition affecting the skin and mucous membranes. It is slightly different to pemphigoid in that autoantibodies damage the junctions that bond epithelial cells to each other. Again the oral mucosa is invariably affected. Although it is slightly less common than pemphigus it is generally more severe and difficult to treat often necessitating the use of systemic steroids and immunomodulatory drugs. However, the use of electrospun muco-adhesive membranes that uni-directionally deliver potent steroids in a slow release fashion would likely preclude the necessity to deliver these drugs systemically.

*Delivery of local anaesthetics*

Local anaesthetics are used widely eg within dentistry. In order to deliver sufficient local anaesthesia for tooth extraction it is usually necessary to give it by nerve block injection or local infiltration injection. Because the injection itself is painful it is not uncommon to first apply topical local anaesthetic gel to the oral mucosa at the intended site of injection. This is frequently done for children and apprehensive patients. Unfortunately, the gel often makes poor contact with the mucosa so that local anaesthetic penetration is poor and most of the gel becomes dissipated in the mouth. This causes unpleasant numbness around the mouth and also has a very bitter and unpleasant taste. As a result the procedure is often of limited effect. Topical delivery of local anaesthetic via a uni-directional , bioadhesive, electrospun composition (eg a drug delivery patch) would result in better localisation and penetration of the local anaesthetic, and thus better efficacy, as well as limiting the adverse effects of widespread numbness and bad taste. The composition would only need a short attachment time or if sterile could be left in place and the injection given through the composition.

An effective bioadhesive, local anaesthetic composition eg in the form of a drug delivery patch, could potentially provide sufficient analgesia for many types of routine dentistry on upper teeth —where infiltration local anaesthesia is usually given, or where procedures are relatively minor.

Local anaesthetic is also extensively used in the mouth for soft tissue surgery including gingival surgery, biopsies etc. Again infiltration anaesthesia is usually given in these situations and it is likely that efficient local anaesthesia could

be obtained in these situations, because bone penetration of the local anaesthetic agent is not required, simply by using a uni-directional, bioadhesive, electrospun local anaesthetic drug composition.

The most obvious local anaesthetic to use in this situation would be lignocaine (lidocaine) hydrochloride although articaine would be a possible alternative. The incorporation of adrenaline as occurs in many local anaesthetic injection solutions may be beneficial in causing local vasoconstriction and thereby enhancing and prolonging the effect of the local anaesthetic agent.

*Treatment of oral mucositis*

Radiotherapy and chemotherapy for cancers are associated with serious side effects. One of the worst is the oral mucositis that occurs. This results in extensive sloughing and ulceration of the oral mucosa. The resulting pain and discomfort often makes eating and drinking impossible and requires the use of narcotic analgesics. Frequently, the cancer treatment has to be abandoned or reduced because of the severity and distress caused by oral mucositis. Currently there is no effective preventative or curative measures. However, the use of bioadhesive wound dressings in accordance with the present invention as discussed above would be helpful in their own right but the inclusion of a local analgesic e.g. benzidamine hydrochloride could be even more effective at alleviating pain. Benzidamine hydrochloride mouthwashes do provide symptomatic relief but their effect is very short lived. This could be extended and enhanced by a composition of the invention, which provides uni-directional delivery to the affected mucosa from the composition eg in the form of a drug delivery patch.

Also recent research has shown that the use of an adrenaline mouthwash before radio- or chemotherapy treatment sessions can help prevent oral mucositis. This appears to be because the vasoconstriction induced in the subepithelial blood vessels by the adrenaline reduces the toxic bystander effects of the treatment on the oral mucosa. Unfortunately, the short contact time with the mucosa that occurs with mouthwash delivery and indiscriminate application to all mucosal sites means that drug delivery is inefficient and systemic side effects of the adrenaline more likely to occur. More direct, prolonged and sustained release of adrenaline into susceptible oral mucosal sites using a composition in accordance with the present invention eg a uni-directional, bioadhesive, electrospun drug delivery patch or membrane delivery system would be far more efficient and effective.

*Delivery of drugs into the oral cavity*

Instead of using electrospun compositions (eg in the form of membranes or patches) to deliver drugs uni-directionally into the oral mucosa to which they are attached as a wound dressing, it is also possible to design composition that adhere to the oral mucosa but deliver drugs into the oral cavity. These can be used to treat more widespread problems in the mouth e.g. oral candidiasis or to slowly deliver drugs to the throat, oesophagus and upper GI tract.

The main advantage of such systems is the ability of the composition (eg in the form of a membrane or a patch) to act as a drug reservoir and slowly but continuously release the drug into the mouth.

(i) Oral candidiasis. This is a common fungal infection of the mouth. It is particularly common in those who wear dentures, those who smoke or have a high sugar intake, those with diabetes or are immunocompromised and those who are taking antibiotics or immunosuppressant treatments including steroids. There are several antifungal drugs that would be effective and safe for treating oral fungal infections (although several are no longer available as oral preparations). However, they all need frequent application because they are rapidly lost from the oral cavity due to swallowing. The main advantage of a composition in accordance with the present invention (eg in the form of a membrane or a patch delivery system) would be the possibility of providing a slow and continual release of drug into the oral cavity. The drugs likely to be most effective and safe would be nystatin and amphotericin. Although the azole antifungals are very effective the risk of systemic absorption and the potential to interact with other drugs means they are likely to have a worse safety profile.

(ii) Drugs can also be delivered to the throat e.g. antiseptics, analgesics and local anaesthetics for treating sore throats colds etc. or to the oesophagus and stomach e.g. antacids, proton pump inhibitors etc or even systemically via the GI tract. The main advantage being the possibility for slow and continuous drug delivery.

*Systemic delivery of drugs across the oral mucosa*

Although the electrospun fibres and compositions according to the invention primarily are intended for local treatment of the skin or mucosa, it is contemplated that electrospun fibres or compositions made in accordance with the present invention but comprising a drug substance that is intended for delivery into the systemic circulation may be suitable for application to the oral mucosa, but for systemic administration through the oral mucosa.

The oral mucosa is readily accessible is more permeable than skin and better supplied with blood vessels. It

also has the advantage that drugs delivered across the oral mucosa and into the circulation avoid the problem of first pass metabolism in the liver. This means that drugs that need rapid administration, including some emergency drugs, and some drugs that would otherwise need to be delivered by injection or would be inactivated in the liver can be more effectively administered across the oral mucosa. Electrospun adhesive drug delivery compositions that uni-directionally deliver such drugs across the oral mucosa can be very effective. They can be used to deliver emergency drugs in the unconscious patients or where injections not possible e.g. where suitably trained staff are not available.

(i) Emergency drug administration: Drugs commonly delivered across the oral mucosa include:

a. Glyceryl trinitrate - This is usually given in the form of a sublingual (under the tongue) spray or quickly dissolving tablet to treat episodes of angina (chest pains). However, the speed of delivery is such that it often causes very severe headache due to the over quick dilatation of cerebral as well as cardiac blood vessels and may need to be repeated several times. Transmucosal delivery of glyceryl trinitrate in a more controlled fashion from a bioadhesive composition eg in the form of a membrane or a patch could produce a smoother and longer duration of dosage and avoid such problems.

b. Aspirin — is often delivered across the oral mucosa in heart attack and stroke patients, particularly when unconscious, in order to reduce thrombosis and worsening of the condition. This is usually achieved by placing a soluble aspirin tablet in the buccal sulcus (between the gums and the inside of the cheek) and allowing it to dissolve. However, much of the drug is lost into the oral cavity rather than accurately delivered across the oral mucosa. Again a more controlled, more directed and longer duration of trans-mucosal delivery could be achieved using the formulation principle in accordance with the present invention, eg in the form of a a bioadhesive electrospun drug delivery patch.

c. Midazolam — is very effective at halting epileptic fits, particularly when they are prolonged or recurring. Although normally given by intravenous injection, this can be very difficult in a fitting patient. So more recently it has been recommended that midazolam solution is simply placed between the cheek and the gums or under the tongue as it rapidly crosses the oral mucosa to enter the circulation and abort fitting. Delivery by this route is uncertain with much of the drug being lost or swallowed. Again, a more controlled, more directed and longer duration of trans-mucosal delivery could be achieved using a composition in accordance with the present invention eg as a bioadhesive electrospun drug delivery patch.

(ii) Delivery of narcotic analgesics. Narcotic (opioid) analgesics are widely used for the treatment of severe and intractable pain particularly cancer related pain and for management of post-operative and trauma related pain (including battlefield injuries). The main problem is that most opioid analgesics need to be given by injection with frequent repeat doses by injection because they are rapidly metabolised in the liver. Some opioid analgesics are now available in patch form for transdermal delivery or sprays for trans nasal delivery but trans mucosal delivery via electrospun bioadhesive oral compositions, eg patches, offers considerable advantages. Including, slower, more controlled and more sustained drug delivery. More effective drug penetration into the circulation than with skin patches as well as the avoidance of first pass metabolism in the liver. Drugs likely to provide good candidates for this approach include: morphine, pethidine, buprenorphine and fentanyl.

**Legends to figures**

**Figure 1A** shows electrospun fibres containing 2.5, 5 or 10 wt% Eudragit® RS100
**Figure 1B** shows electrospun fibres containing 5, 10 or 15 wt% Eudragit® RS100 after exposure to water
**Figure 2** shows SEM micrographs of fibres containing 10 wt% dextran or 10 wt% PEO
**Figure 3** shows SEM micrographs of fibres coated with 10 wt% PCL before or after baking

**Materials**

- Polyvinylpyrrolidone with Mw 1,000,000 - 1,500,000 (PVP). BASF, Germany.
- Eudragit RS100 (RS100). Evonik Industries, Germany.
- Polycaprolactone with Mw 80,000 (PCL). Sigma Aldrich, UK.
- Dextrans with Mw 500,000 (DEX5). Pharmacosmos, Denmark.
- Polyethylene oxide with Mw 400,000 (PEO4) and Mw 2,000,000 (PEO20). Sigma Aldrich, UK

- Clobetasol propionate analytical standard. Sigma Aldrich, UK.
- Solvent for PVP and RS100: 97 vol% ethanol (Sigma Aldrich, UK) in distilled water.
- Solvent for PCL: 90/10 vol/vol blend of dichloromethane/dimethylformamide (Fisher Scientific, UK).
- Baking paper or tin foul as the substrate used to produce the electrospun layers.

**Methods**

**Determination of solubility of bioadhesive substances or hydrophilic fibre-forming polymer**

The solubility of the bioadhesive substances or hydrophilic fibre-forming polymers was determined using a method recommended by the European Pharmacopoeia 5.0 (Section 5.11, p. 565).

The European Pharmacopoeia uses the following terms to define the solubility of a substance in a particular solvent (Section 1.4, p. 7):

| Descriptive term | Approximate volume of solvent in mL per g of solute | | | |
|---|---|---|---|---|
| Very soluble | Less than | 1 | | |
| Freely soluble | From | 1 | To | 10 |
| Soluble | From | 10 | To | 20 |
| Sparingly soluble | From | 30 | To | 100 |
| Slightly soluble | From | 100 | To | 1000 |
| Very slightly soluble | From | 1000 | To | 10000 |
| Practically insoluble | More than | | | 10000 |

The experimental method used to determine the solubility of a substance is described in the following:
*Dissolving procedure:* Shake tube (1 min) and place in a constant temperature device at a temperature of 25±0.5 °C for 15 min. If the substance is not completely dissolved, repeat the shaking (1 min) and place the tube in the constant temperature device for 15 min.

*Method:*

1) Weigh 100 mg of finely powdered substance in a stoppered tube (16 mm in internal diameter and 160 mm long), add 0.1 ml of the solvent and proceed as described under Dissolving Procedure. If the substance is completely dissolved, it is *very soluble.*
2) If the substance is not completely dissolved, add 0.9 ml of the solvent and proceed as described under Dissolving Procedure. If the substance is completely dissolved, it is *freely soluble.*
3) If the substance is not completely dissolved, add 2.0 ml of the solvent and proceed as described under Dissolving Procedure. If the substance is completely dissolved, it is *soluble.*
4) If the substance is not completely dissolved, add 7.0 ml of the solvent and proceed as described under Dissolving Procedure. If the substance is completely dissolved, it is *sparingly soluble.*
5) If the substance is not completely dissolved, weigh 10 mg of finely powdered substance in a stoppered tube, add 10.0 ml of the solvent and proceed as described under Dissolving Procedure. If the substance is completely dissolved, it is *slightly soluble.*
6) If the substance is not completely dissolved, weigh 1 mg of finely powdered substance in a stoppered tube, add 10.0 ml of the solvent and proceed as described under Dissolving Procedure. If the substance is completely dissolved, it is *very slightly soluble.*

## Examples

### Example 1

**Preparation of fibres - basic description of the preparation of polymeric solutions and suspension, and eletro spinning conditions**

In order to produce the fibres, polymeric dispersions were prepared by adding the different components to the solvent and then stirring overnight on a magnetic stirrer. The fibre-forming hydrophilic polymer(s) was/were soluble in the solvent, whereas the bioadhesive substance has a lower solubility and is mainly present as solid material.
Fibres were spun using the following electrospinning conditions:

- 15 gauge needle
- Voltage = 16 kV
- Distance = 19 cm
- Flow rate = 5 ml/h

Fibres were also spun with a content of a drug substance. In these cases, the drug substance is dissolved/dispersed in the solvent together with the bioadhesive substance and/or hydrophilic polymers.

### Example 2

**Preparation of electrospun fibres containing PVP and Eudragit® RS100**

PVP and Eudragit® RS100 are dissolved in ethanol 97 vol% and subjected to electrospinning as described herein. Polymer blends were homogeneous and no phase separation was observed at any point. The results obtained are:

| PVP (wt%) | Eudragit RS100 (wt%) | 97% ethanol (wt%) | Outcomes |
|---|---|---|---|
| 10 | 2.5 | 87.5 | All compositions could be electrospun. Fibres became more rigid as the proportion of RS100 increased. Consequently, the resulting material exhibited an increased compactness and a decreased porosity. The solubility of the material decreased significantly with the addition of RS100, even as little as 2.5 wt%. Significant shrinkage of samples as water is absorbed. |
| | 3 | 87 | |
| | 4 | 86 | |
| | 5 | 85 | |
| | 6 | 84 | |
| | 7 | 83 | |
| | 8 | 82 | |
| | 10 | 80 | |
| | 15 | 75 | |

Figure 1A and 1B show SEM micrographs of all compositions.
The above-mentioned examples were repeated with a content of 0.05-1% w/w of a drug substance.

### Example 3

**Addition of bioadhesive substance - preliminary study of addition of bioadhesive stubstance to fibres maintaining concentrations of PVP and Eudragit RS100 fixed**

Various concentrations of particulate dextrans (DEX) and poly(ethylene oxide) (PEO) were added to PVP/RS100 solutions in order to increase bioadhesive properties of the electrospun materials.

| | Content (wt%) | | | | | | |
|---|---|---|---|---|---|---|---|
| | 5 | 10 | 20 | 30 | 40 | 50 | 60 |
| Dextran | Yes | Yes | Yes | Yes | Yes, partially | No | - |
| Poly(ethylene oxide) | Yes | Yes | Yes | Yes, partially | No | - | - |

Figure 2 shows SEM micrographs of the compositins with 10% dextran and 10% PEO, respectively.

**Example 4**

**Preparation of fibres using dextrans as bioadhesive substance - increasing concentations of Eudragit® RS100**

**Aim:** To demonstrate the fabrication of bioadhesive fibres containing dextran particles as bioadhesive substance.

A series of dispersions were prepared, and fibres were produced following the method previously described. Dex 500,000 could easily be substituted with Dex 2,000,000 and give suitable results (fibres)

| PVP | RS100 | DEX5 | Solvent | Fibres |
|---|---|---|---|---|
| 10 wt% | 5 wt% | 5 wt% | 80.00 wt% | Yes |
| 10 wt% | 5 wt% | 10 wt% | 75.00 wt% | Yes |
| 10 wt% | 5 wt% | 20 wt% | 65.00 wt% | Yes |
| 10 wt% | 10 wt% | 5 wt% | 75.00 wt% | Yes |
| 10 wt% | 10 wt% | 10 wt% | 70.00 wt% | Yes |
| 10 wt% | 10 wt% | 20 wt% | 60.00 wt% | Yes |
| 10 wt% | 15 wt% | 5 wt% | 70.00 wt% | Yes |
| 10 wt% | 15 wt% | 10 wt% | 65.00 wt% | Yes |
| 10 wt% | 15 wt% | 20 wt% | 55.00 wt% | Yes |

The above-mentioned examples were repeated with a content of 0.05-1% w/w of a drug substance.

**Example 5**

**Preparation of fibres using polyethylene oxide as bioadhesive substance - increasing concentrations of Eudragit® RS100**

**Aim:** To demonstrate the fabrication of bioadhesive fibres containing polyethylene oxide particles as bioadhesive substance.

A series of dispersions were prepared, and fibres were produced following the method previously described. PEO with a molecular weight of 400,000 was used, but could easily be substituted with PEO 2,000,000 and form fibres.

| PVP | RS100 | PEO4 | Solvent | Fibres |
|---|---|---|---|---|
| 10 wt% | 5 wt% | 5 wt% | 80.00 wt% | Yes |
| 10 wt% | 5 wt% | 10 wt% | 75.00 wt% | Yes |
| 10 wt% | 5 wt% | 20 wt% | 65.00 wt% | Yes |
| 10 wt% | 10 wt% | 5 wt% | 75.00 wt% | Yes |
| 10 wt% | 10 wt% | 10 wt% | 70.00 wt% | Yes |
| 10 wt% | 10 wt% | 20 wt% | 60.00 wt% | Yes |
| 10 wt% | 15 wt% | 5 wt% | 70.00 wt% | Yes |
| 10 wt% | 15 wt% | 10 wt% | 65.00 wt% | Yes |
| 10 wt% | 15 wt% | 20 wt% | 55.00 wt% | Yes |

The above-mentioned examples were repeated with a content of 0.05-1% w/w of a drug substance.

**Example 6**

**Preparation of fibres containing a drug substance and a bioadhesive substance**

To demonstrate the fabrication of bioadhesive fibres containing the drug clobetasol propionate, and dextran or polyethylene oxide particles as bioadhesive substances.

A series of dispersions were prepared, and fibres were produced following the method previously described.

| PVP | RS100 | DEX5 | PEO20 | Drug | Solvent | Fibres |
|---|---|---|---|---|---|---|
| 10 wt% | 0 wt% | 10 wt% | - | 0.05 wt% | 79.95 wt% | Yes |
| 10 wt% | 0 wt% | - | 10 wt% | 0.05 wt% | 79.95 wt% | Yes |
| 10 wt% | 5 wt% | 10 wt% | - | 0.05 wt% | 74.95 wt% | Yes |
| 10 wt% | 5 wt% | - | 10 wt% | 0.05 wt% | 74.95 wt% | Yes |
| 10 wt% | 10 wt% | 10 wt% | - | 0.05 wt% | 69.95 wt% | Yes |
| 10 wt% | 10 wt% | - | 10 wt% | 0.05 wt% | 69.95 wt% | Yes |
| 10 wt% | 15 wt% | 10 wt% | - | 0.05 wt% | 64.95 wt% | Yes |
| 10 wt% | 15 wt% | - | 10 wt% | 0.05 wt% | 64.95 wt% | Yes |

**Example 7A**

**Compositions with hydrophobic backing layer**

Electrospun fibres as described in Example 2 was coated with a backing layer. Figure 3 shows the results before and after baking (subjecting the backing layer to a temperature of at least 60°C and at the most 200 °C.

**Example 7B**

**Preparation of a two-layered composition made of fibres containing clobetasol propionate on hydrophobic fibres**

**Aim:** To demonstrate the fabrication of two-layered bioadhesive fibres, made of a layer containing the drug clobetasol propionate and a hydrophobic and non adhesive backing layer.

**Method of fabrication:**

- First, a layer of PCL was produced on tin foil.
- Once the PCL was partially dry, a layer of the drug containing solution was electrospinned on top. Alternatively, a film or fibres of PCL was made upon which the electrospun fibres containing clobetasol were provided.

All fibres were then left to dry for a prolonged amount of time (1 hour minimum) at room temperature.

**Compositions:** Same as in example 6

**Example 8**

***In vitro* testing of adhesive properties of fibres, on plastic**

**Aim:** To demonstrate the bioadhesive properties of the fibres *in vitro.*

A series of two-layered compositions of the fibres were prepared following the methods described previously. The PCL layer is either casted or electrospun.

| PVP | RS100 | DEX5 | PEO20 | Solvent | Fibres |
|---|---|---|---|---|---|
| 10 wt% | 0 wt% | - | - | 90 wt% | Yes |
| 10 wt% | 10 wt% | - | - | 80 wt% | Yes |
| 10 wt% | 0 wt% | 10 wt% | - | 80 wt% | Yes |
| 10 wt% | 0 wt% | - | 10 wt% | 80 wt% | Yes |
| 10 wt% | 10 wt% | 10 wt% | - | 70 wt% | Yes |
| 10 wt% | 10 wt% | - | 10 wt% | 70 wt% | Yes |

**Method of study:**

- Samples of dimensions 1.5 × 1 cm were cut from each fibre composition and were applied to a Petri dish that had been previously wetted with 1 mL of water.
- Samples were applied by pressing on the surface of the PCL backing layer with a finger for a minimum time of 5 seconds.
- The samples were then covered with artificial saliva (Volume = 3-5 mL), and the petri dishes were placed on a rocker tray set at a speed of 50 rpm.
- The dishes were continuously observed in order to determine the time at which the backing layer detached from the plastic.

Study terminated after 1 hour.

**Example 9**

**Fibre compositions according to the invention - in vitro studies using porcine mucosa**

Electrospun fibres containing Eudragit® RS100 (ammonio methacrylate copolymer type B; molecular weight 32,000 g/mol) and PVP were prepared as previously described. Compositions were made containing: 10% w/w PVP, from 2.5-20% w/w Eudragit® RS100, and from 5-10% w/w of either dextran or PEO. The results are shown below.

Similar compositions, but substituting Eudragit® RS 100 with Eudragit L100-55 (methacrylic acid- ethyl acrylate copolymer (1:1) Type A) were also prepared.

In order to test the fibre compositions ability to remain on a mucosal surface, the fibre compositions were applied to porcine mucosa with force for 1 minute in order to ensure bioadhesion. Then the mucosa was submerged in simulated saliva with a rotation of 40 rpm. The residence time was measured as the time it takes for 50% or more of the composition to detach from the oral mucosa. The results are shown in below.

| **Materials** | **Components (concentration in solution before electrospinning)** | | | | **In vitro time before 50% detachment (min)** |
|---|---|---|---|---|---|
| | PCL | PVP | Eudragit RS 100 | Dextran | |
| PCL only | 10 wt% | - | - | - | 2 |
| PVP+PCL | 10 wt% | 10 wt% | - | - | 30 |
| 10% Dextran | 10 wt% | 10 wt% | - | 10 wt% | 130 |
| Patch 1 | 10 wt% | 10 wt% | 5 wt% | - | 320 |
| Patch 2 | 10 wt% | 10 wt% | 5 wt% | 10 wt% | 380 |
| Patch 3 | 10 wt% | 10 wt% | 10 wt% | 10 wt% | 380 |

- PCL was dissolved in a blend of dichloromethane (DCM) and dimethylformamide (DMF) with proportion 90:10 vol% DCM:DMF. The green colour was added using a conventional food dye dissolved in the solvents before fabrication of the material.

- PVP and Eudragit RS100 were dissolved in 97 vol% ethanol in distilled water.

**Example 10**

***In vivo* adhesion testing of compositions**

Aim: To demonstrate the bioadhesive properties of the fibres *in vivo*
A series of two-layered compositions of the fibres were prepared following the methods described previously.

| PVP | RS100 | DEX5 | PEO20 | Solvent | Fibres |
|---|---|---|---|---|---|
| 10 wt% | 0 wt% | - | - | 90 wt% | Yes |
| 10 wt% | 10 wt% | - | - | 80 wt% | Yes |
| 10 wt% | 0 wt% | 10 wt% | - | 80 wt% | Yes |
| 10 wt% | 0 wt% | - | 10 wt% | 80 wt% | Yes |
| 10 wt% | 0 wt% | 20 wt% | - | 70 wt% | Yes |
| 10 wt% | 0 wt% | - | 20 wt% | 70 wt% | Yes |
| 10 wt% | 10 wt% | 10 wt% | - | 70 wt% | Yes |
| 10 wt% | 10 wt% | - | 10 wt% | 70 wt% | Yes |
| 10 wt% | 10 wt% | 20 wt% | - | 60 wt% | Yes |
| 10 wt% | 10 wt% | - | 20 wt% | 60 wt% | Yes |

**Method of analysis:**

- Samples of dimensions 1.5 × 1 cm were cut from each fibre composition and were tested on the tongue of a volunteer.

The volunteer then was asked to evaluate the strength of the adhesion from 0 to 5, where 0 indicates that there is no adhesion and 5 indicates a strong adhesion of the fibres.

**Example 11**

***In vitro* drug release testing of fibres**

**Aim:** To demonstrate the release of the drug clobetasol propionate contained within electrospun fibres
**Compositions:** Same as in example 4.
Composition of Green's cell culture medium - Dulbecco's Modified Eagle's Medium : Ham's F12 medium in a 3:1 (v/v) ratio supplemented with 10 % (v/v) FCS

- 0.1 μM cholera toxin
- 10 ng/ml of epidermal growth factor (EGF)

- 0.4 μg/ml hydrocortisone
- 0.18 mM adenine
- 5 μg/ml insulin
- 5 μg/ml transferrin
- 2 mM glutamine
- 0.2 μM triiodothyronine
- 0.625 μg/ml amphotericin B
- 100 IU/ml penicillin
- 100 μg/ml streptomycin

**Method of analysis:**

- Samples of dimensions 1.2 × 1.2 cm were cut from each fibre composition and were placed in vials containing 5 mL of Green's cell culture medium.
- All vials were incubated at 37°C for 30, 60 and 120 minutes.
- At those time points, 1.5 mL of cell culture media were removed from each vial
- The concentration of clobetasol propionate released from the fibres was measured using high-performance liquid chromatography (HPLC).

**Conclusions:** The drug clobetasol propionate was successfully incorporated into the fibres and was released after immersion in cell culture media. The addition of RS100 may result in slowed release.

## Claims

**1.** Electrospun fibres containing:

i) a first and a second hydrophilic fibre-forming polymer that is soluble in a hydrophilic solvent,
ii) a bioadhesive substance that is slightly soluble in said hydrophilic solvent,
iii) a drug substance,

wherein the first hydrophilic polymer has a solubility in water at 37°C that is at least 10 times greater than the solubility in water at 37°C of the second hydrophilic fibre-forming polymer, and wherein the bioadhesive substance is present in solid form.

**2.** Electrospun fibers according to claim 1, wherein the concentration of the first hydrophilic fibre-forming polymer in the fibres is in a range of from 10 to 60% w/w, notably from 20 to 50% w/w or from 25 to 45% w/w, and the concentration of the second hydrophilic fibre-forming polymer in the fibres is in a range of from 10 to 60% w/w, notably from 20 to 50% w/w or from 25 to 45% w/w.

**3.** Electrospun fibres according to claim 1, wherein said first and the second hydrophilic fibre-forming polymer has a solubility in said hydrophilic solvent of 3 g/100 ml or more at 25 °C or 10 g/100 ml or more at 25 °C.

**4.** Electrospun fibres according to any of the preceding claims, wherein said hydrophilic solvent is selected from ethanol, or ethanol-water mixtures.

**5.** Electrospun fibres according to claim 3, wherein said ethanol-water mixtures contain 20% v/v water or less or 10% v/v water or less.

**6.** Electrospun fibres according to claim 3 or 4, wherein said ethanol-water mixtures contain 5% v/v water or less such as 3% v/v water of less.

**7.** Electrospun fibres according to any of the preceding claims, wherein said first hydrophilic polymer has a solubility in water that is at least 50 times greater than the water solubility of said second hydrophilic polymer in water, both measured at 37°C.

8. Electrospun fibres according to any of the preceding claims, wherein said first hydrophilic polymer has a solubility in water that is at least 100 times greater or at least 500 times greater than the water solubility of said second hydrophilic polymer in water, both measured at 37°C.

9. Electrospun fibres according to any of the preceding claims, wherein the bioadhesive substance is at the most very slightly soluble in said solvent selected from ethanol, or ethanol-water mixtures at a temperature of 25 °C.

10. Electrospun fibres according to any of the preceding claims, wherein the bioadhesive substance has a solubility of at the most 0.1% w/w in said solvent selected from ethanol,ethanol-water mixtures at a temperature of 25 °C.

11. Electrospun fibres according to any of the preceding claims, wherein the bioadhesive substance has a solubility of at the most 0.01% w/w in said solvent selected from ethanol, ethanol-water mixtures at a temperature of 25 °C.

12. Electrospun fibres according to any of the preceding claims, wherein at least 90% w/w of the bioadhesive substance is present in solid form.

13. Electrospun fibres according to any of the preceding claims, wherein said first hydrophilic polymer is selected from polyvinylpyrrolidone (PVP), hydroxypropylcellulose, and mixtures thereof.

14. Electrospun fibres according to any of the preceding claims, wherein said second hydrophilic polymer is selected from ethylcellulose, acrylates and acrylic copolymers (Eudragit®), and mixtures thereof

15. Electrospun fibres according to any of the preceding claims, wherein the first hydrophilic polymer is PVP and the second hydrophilic polymer is an acrylic copolymer such as an Eudragit®.

16. Electrospun fibres according to any of the preceding claims, wherein the bioadhesive substance is selected from dextrans, polyethylene oxides (PEOs), alginate, tragacanth, carrageenan, pectin, gelatin, guar, xanthan, gellan, methylcellulose, hydroxypropylmethylcellulose (HPMC), polyvinylalcohol (PVA), polymers of acrylic acids (PAA derivatives), chitosan, lectins, thiolated polymers, polyoxo WSRA, PAA-co-PEG (PEG is polyethylene glycol), and mixtures thereof.

17. Electrospun fibres according to any of the preceding claims, wherein the bioadhesive substance is dextran having an average molecular weight of from 400,000 Da to 2,000,000 Da.

18. Electrospun fibres according to any of the preceding claims, wherein the bioadhesive substance is dextran having an average molecular weight of about 2,000,000 Da.

19. Electrospun fibres according to any of claims 1-16, wherein the bioadhesive substance is polyethylene oxide having an average molecular weight of from 100,000 Da to 4,000,000 Da.

20. Electrospun fibres according to any of claims 1-16, wherein the bioadhesive substance is polyethylene oxide having an average molecular weight of 2,000,000 Da.

21. Electrospun fibres according to any of the preceding claims, wherein the weight ratio between the bioadhesive substance and the hydrophilic fibre-forming polymers in the fibres is in a range of from 0.1 to 10.

22. Electrospun fibres according to any of the preceding claims, wherein the drug substance is selected from drug substances, which are indicated for treatment of a disease of the skin or mucosa.

23. Electrospun fibres according to any of the preceding claims, wherein the drug substance is selected from drug substances, which are indicated for treatment of a disease in the oral or vaginal cavity.

24. Electrospun fibres according to claim 23, wherein the drug substance is selected from drug substances, which are indicated for local treatment of a disease in the oral cavity.

25. Electrospun fibres according to any of the preceding claims, wherein the water content is at the most about 5% w/w.

26. Electrospun fibres according to any of the preceding claims for use in medicine.

**27.** Electrospun fibres according to any of the preceding claims for use in the treatment of diseases of the oral or vaginal cavity.

**28.** A composition comprising electrospun fibres as defined in any of claims 1-27.

**29.** A composition according to claim 28, wherein the concentration of the electrospun fibres in the composition is from 70 to 100% w/w.

**30.** A composition according to claim 28 or 29 in the form of a layered composition.

**31.** Electrospun fibres according to any one of claims 1-27 or a composition according to any one of claims 28-30, wherein an outer surface of said electrospun fibres or composition is provided with a coating.

**32.** Electrospun fibres or a composition according to claim 31, wherein the coating is water-impermeable.

**33.** Electrospun fibres or a composition according to claim 31 or 32, wherein the coating comprises carbothane, polycaprolactone or polyethylene-co-vinyl acetate, or mixtures thereof.

**34.** Electrospun fibres as defined in any of claims 1-27 or a composition as defined in any of claims 28-33 for use in medicine.

**35.** Electrospun fibres according to any of claim 1-27 or a composition as defined in any of claims 28-35 for use in the treatment of diseases of the oral cavity.

**36.** A kit comprising

i) electrospun fibres or a composition as defined in any of the preceding claims, and
ii) an applicator for applying the composition in the oral cavity.

**37.** A method for preparing electrospun fibres as defined in any of claims 1-27, the method comprising

i) dissolving the first and the second hydrophilic polymer in a solvent selected from ethanol, or ethanol-water mixtures,
ii) suspending the bioadhesive substance in the resulting solution from step i),
iii) if relevant, adding the drug substance to the resulting dispersion from step ii),
iv) electrospinning the resulting mixture from step ii) or, if relevant step iii),

wherein said first and second hydrophilic polymer is soluble in said solvent, and said bioadhesive substance is slightly soluble or less in said solvent,
to obtain electrospun fibres, wherein at least 90% w/w of the bioadhesive substance is present in solid form.

**38.** A method for preparing electrospun fibres according to any of claims 1-27, the method comprising

i) dissolving the first and second hydrophilic polymer in a solvent selected from ethanol, water or mixtures thereof to obtain a first solution,
ii) if relevant, dissolving or suspending the drug substance in said solution to obtain a mixture,
iii) suspending the bioadhesive substance in the solvent to obtain a dispersion,
iv) dual-electrospinning the mixture from step i), or if relevant step ii) and the dispersion from iii),

wherein said first and second hydrophilic polymer is soluble in said solvent, and said bioadhesive substance is slightly soluble in said solvent,
to obtain electrospun fibres, wherein at least 90% w/w of the bioadhesive substance is present in solid form.

**39.** A method according to claim 37 or 38 further comprising a step of coating an outer surface of the fibres with a hydrophobic polymer.

**40.** A method according to claim 39, wherein the coated fibres are subject to heating to melt or soften the hydrophobic polymer.

**Patentansprüche**

1. Elektrogesponnene Fasern, die Folgendes enthalten:

   i) ein erstes und ein zweites hydrophiles, faserbildendes Polymer, das in einem hydrophilen Lösungsmittel löslich ist,
   ii) eine bioadhäsive Substanz, die in dem genannten hydrophilen Lösungsmittel geringfügig löslich ist,
   iii) eine Arzneimittelsubstanz,

   wobei das erste hydrophile Polymer eine Löslichkeit in Wasser bei 37°C hat, die wenigstens 10 Mal höher ist als die Löslichkeit des zweiten hydrophilen, faserbildenden Polymers in Wasser bei 37°C, und wobei die bioadhäsive Substanz in fester Form vorliegt.

2. Elektrogesponnene Fasern nach Anspruch 1, wobei die Konzentration des ersten hydrophilen, faserbildenden Polymers in den Fasern in einem Bereich von 10 bis 60 Gew.-%, insbesondere 20 bis 50 Gew.-% oder 25 bis 45 Gew.-%, liegt und die Konzentration des zweiten hydrophilen, faserbildenden Polymers in den Fasern in einem Bereich von 10 bis 60 Gew.-%, insbesondere 20 bis 50 Gew.-% oder 25 bis 45 Gew.-% liegt.

3. Elektrogesponnene Fasern nach Anspruch 1, wobei das genannte erste und zweite hydrophile, faserbildende Polymer eine Löslichkeit in dem genannten hydrophilen Lösungsmittel von 3 g/100 ml oder mehr bei 25°C oder 10 g/100 ml oder mehr bei 25°C hat.

4. Elektrogesponnene Fasern nach einem der vorherigen Ansprüche, wobei das genannte hydrophile Lösungsmittel aus Ethanol oder Ethanol-Wasser-Gemischen ausgewählt ist.

5. Elektrogesponnene Fasern nach Anspruch 3, wobei die genannten Ethanol-Wasser-Gemische 20 Vol.-% Wasser oder weniger oder 10 Vol.-% Wasser oder weniger enthalten.

6. Elektrogesponnene Fasern nach Anspruch 3 oder 4, wobei die genannten Ethanol-Wasser-Gemische 5 Vol.-% Wasser oder weniger, wie z.B. 3 Vol.-% Wasser oder weniger, enthalten.

7. Elektrogesponnene Fasern nach einem der vorherigen Ansprüche, wobei das genannte erste hydrophile Polymer eine Löslichkeit in Wasser hat, die wenigstens 50 Mal höher ist als die Löslichkeit des genannten zweiten hydrophilen Polymers in Wasser, jeweils bei 37°C gemessen.

8. Elektrogesponnene Fasern nach einem der vorherigen Ansprüche, wobei das genannte erste hydrophile Polymer eine Löslichkeit in Wasser hat, die wenigstens 100 Mal höher oder wenigstens 500 mal höher ist als die Löslichkeit des genannten zweiten hydrophilen Polymers in Wasser, jeweils bei 37°C gemessen.

9. Elektrogesponnene Fasern nach einem der vorherigen Ansprüche, wobei die bioadhäsive Substanz in dem genannten Lösungsmittel, ausgewählt aus Ethanol oder Ethanol-Wasser-Gemischen, bei einer Temperatur von 25°C höchstens sehr geringfügig löslich ist.

10. Elektrogesponnene Fasern nach einem der vorherigen Ansprüche, wobei die bioadhäsive Substanz eine Löslichkeit von höchstens 0,1 Gew.-% in dem genannten Lösungsmittel, ausgewählt aus Ethanol, Ethanol-Wasser-Gemischen, bei einer Temperatur von 25°C hat.

11. Elektrogesponnene Fasern nach einem der vorherigen Ansprüche, wobei die bioadhäsive Substanz eine Löslichkeit von höchstens 0,01 Gew.-% in dem genannten Lösungsmittel, ausgewählt aus Ethanol, Ethanol-Wasser-Gemischen, bei einer Temperatur von 25°C hat.

12. Elektrogesponnene Fasern nach einem der vorherigen Ansprüche, wobei wenigstens 90 Gew.-% der bioadhäsiven Substanz in fester Form vorliegen.

13. Elektrogesponnene Fasern nach einem der vorherigen Ansprüche, wobei das genannte erste hydrophile Polymer aus Polyvinylpyrrolidon (PVP), Hydroxypropylcellulose und Gemischen davon ausgewählt ist.

14. Elektrogesponnene Fasern nach einem der vorherigen Ansprüche, wobei das genannte zweite hydrophile Polymer

aus Ethylcellulose, Acrylaten und Acrylcopolymeren (Eudragit®) und Gemischen davon ausgewählt ist.

**15.** Elektrogesponnene Fasern nach einem der vorherigen Ansprüche, wobei das erste hydrophile Polymer PVP ist und das zweite hydrophile Polymer ein Acrylcopolymer wie z.B. Eudragit® ist.

**16.** Elektrogesponnene Fasern nach einem der vorherigen Ansprüche, wobei die bioadhäsive Substanz ausgewählt ist aus Dextranen, Polyethylenoxiden (PEOs), Alginat, Traganth, Carrageen, Pektin, Gelatine, Guar, Xanthan, Gellan, Methylcellulose, Hydroxypropylmethylcellulose (HPMC), Polyvinylalkohol (PVA), Polymeren von Acrylsäuren (PAA-Derivate), Chitosan, Lektinen, thiolierten Polymeren, Polyoxo WSRA, PAA-co-PEG (PEG ist Polyethylenglykol) und Gemischen davon.

**17.** Elektrogesponnene Fasern nach einem der vorherigen Ansprüche, wobei die bioadhäsive Substanz Dextran mit einer durchschnittlichen Molekülmasse von 400.000 Da bis 2.000.000 Da ist.

**18.** Elektrogesponnene Fasern nach einem der vorherigen Ansprüche, wobei die bioadhäsive Substanz Dextran mit einer durchschnittlichen Molekülmasse von etwa 2.000.000 Da ist.

**19.** Elektrogesponnene Fasern nach einem der Ansprüche 1-16, wobei die bioadhäsive Substanz Polyethylenoxid mit einer durchschnittlichen Molekülmasse von 100.000 Da bis 4.000.000 Da ist.

**20.** Elektrogesponnene Fasern nach einem der Ansprüche 1-16, wobei die bioadhäsive Substanz Polyethylenoxid mit einer durchschnittlichen Molekülmasse von 2.000.000 Da ist.

**21.** Elektrogesponnene Fasern nach einem der vorherigen Ansprüche, wobei das Gewichtsverhältnis zwischen der bioadhäsiven Substanz und den hydrophilen, faserbildenden Polymeren in den Fasern in einem Bereich von 0,1 bis 10 liegt.

**22.** Elektrogesponnene Fasern nach einem der vorherigen Ansprüche, wobei die Arzneimittelsubstanz ausgewählt ist aus Arzneimittelsubstanzen, die zur Behandlung einer Erkrankung der Haut oder Schleimhaut indiziert sind.

**23.** Elektrogesponnene Fasern nach einem der vorherigen Ansprüche, wobei die Arzneimittelsubstanz ausgewählt ist aus Arzneimittelsubstanzen, die zur Behandlung einer Erkrankung der Mund- oder Vaginalhöhle indiziert sind.

**24.** Elektrogesponnene Fasern nach Anspruch 23, wobei die Arzneimittelsubstanz aus Arzneimittelsubstanzen ausgewählt ist, die zur lokalen Behandlung einer Erkrankung der Mundhöhle indiziert sind.

**25.** Elektrogesponnene Fasern nach einem der vorherigen Ansprüche, wobei der Wassergehalt höchstens etwa 5 Gew.-% beträgt.

**26.** Elektrogesponnene Fasern nach einem der vorherigen Ansprüche zur Verwendung in der Medizin.

**27.** Elektrogesponnene Fasern nach einem der vorherigen Ansprüche zur Verwendung bei der Behandlung von Erkrankungen der Mund- oder Vaginalhöhle.

**28.** Zusammensetzung, die elektrogesponnene Fasern nach einem der Ansprüche 1-27 umfasst.

**29.** Zusammensetzung nach Anspruch 28, wobei die Konzentration der elektrogesponnenen Fasern in der Zusammensetzung 70 bis 100 Gew.-% beträgt.

**30.** Zusammensetzung nach Anspruch 28 oder 29 in Form einer geschichteten Zusammensetzung.

**31.** Elektrogesponnene Fasern nach einem der Ansprüche 1-27 oder Zusammensetzung nach einem der Ansprüche 28-30, wobei eine Außenfläche der genannten elektrogesponnenen Fasern oder der Zusammensetzung mit einer Beschichtung versehen ist.

**32.** Elektrogesponnene Fasern oder Zusammensetzung nach Anspruch 31, wobei die Beschichtung wasserundurchlässig ist.

**33.** Elektrogesponnene Fasern oder Zusammensetzung nach Anspruch 31 oder 32, wobei die Beschichtung Carbothan, Polycaprolacton oder Polyethylen-co-vinylacetat oder Gemische davon umfasst.

**34.** Elektrogesponnene Fasern nach einem der Ansprüche 1-27 oder Zusammensetzung nach einem der Ansprüche 28-33 zur Verwendung in der Medizin.

**35.** Elektrogesponnene Fasern nach einem der Ansprüche 1-27 oder Zusammensetzung nach einem der Ansprüche 28-35 zur Verwendung bei der Behandlung von Erkrankungen der Mundhöhle.

**36.** Kit, der Folgendes umfasst:

i) elektrogesponnene Fasern oder eine Zusammensetzung nach einem der vorherigen Ansprüche und
ii) einen Applikator zum Auftragen der Zusammensetzung in der Mundhöhle.

**37.** Verfahren zur Herstellung elektrogesponnener Fasern nach einem der Ansprüche 1-27, wobei das Verfahren Folgendes beinhaltet:

i) Lösen des ersten und zweiten hydrophilen Polymers in einem Lösungsmittel, ausgewählt aus Ethanol oder Ethanol-Wasser-Gemischen,
ii) Suspendieren der bioadhäsiven Substanz in der resultierenden Lösung aus Schritt i),
iii) sofern relevant, Zugeben der Arzneimittelsubstanz zur resultierenden Dispersion aus Schritt ii),
iv) Elektrospinnen des resultierenden Gemischs aus Schritt ii) oder, sofern relevant, Schritt iii), wobei das genannte erste und zweite hydrophile Polymer in dem genannten Lösungsmittel löslich ist und die genannte bioadhäsive Substanz in dem genannten Lösungsmittel geringfügig oder weniger löslich ist,

um elektrogesponnene Fasern zu erhalten, wobei wenigstens 90 Gew.-% der bioadhäsiven Substanz in fester Form vorliegen.

**38.** Verfahren zur Herstellung elektrogesponnener Fasern nach einem der Ansprüche 1-27, wobei das Verfahren Folgendes beinhaltet:

i) Lösen des ersten und zweiten hydrophilen Polymers in einem Lösungsmittel, ausgewählt aus Ethanol, Wasser oder Gemischen davon, um eine erste Lösung zu erhalten,
ii) sofern relevant, Lösen oder Suspendieren der Arzneimittelsubstanz in der genannten Lösung, um ein Gemisch zu erhalten,
iii) Suspendieren der bioadhäsiven Substanz in dem Lösungsmittel, um eine Dispersion zu erhalten,
iv) Doppelelektrospinnen des Gemischs aus Schritt i) oder, sofern relevant, Schritt ii) und der Dispersion aus iii),

wobei das genannte erste und zweite hydrophile Polymer in dem genannten Lösungsmittel löslich sind und die genannte bioadhäsive Substanz in dem genannten Lösungsmittel geringfügig löslich ist,
um elektrogesponnene Fasern zu erhalten, wobei wenigstens 90 Gew.-% der bioadhäsiven Substanz in fester Form vorliegen.

**39.** Verfahren nach Anspruch 37 oder 38, das ferner einen Schritt des Beschichtens einer Außenfläche der Fasern mit einem hydrophoben Polymer beinhaltet.

**40.** Verfahren nach Anspruch 39, wobei die beschichteten Fasern erhitzt werden, um das hydrophobe Polymer zu schmelzen oder zu erweichen.

## Revendications

**1.** Fibres électrofilées contenant :

i) un premier et un second polymères fibrogènes hydrophiles qui sont solubles dans un solvant hydrophile,
ii) une substance bioadhésive qui est légèrement soluble dans ledit solvant hydrophile,
iii) une substance médicamenteuse,

où le premier polymère hydrophile a une solubilité dans l'eau à 37 °C qui est au moins 10 fois plus élevée que la solubilité dans l'eau à 37 °C du second polymère fibrogène hydrophile, et où la substance bioadhésive est présente sous une forme solide.

2. Fibres électrofilées selon la revendication 1, où la concentration en le premier polymère fibrogène hydrophile dans les fibres est dans une plage de 10 à 60 % p/p, notamment de 20 à 50 % p/p ou de 25 à 45 % p/p, et la concentration en le second polymère fibrogène hydrophile dans les fibres est dans une plage de 10 à 60 % p/p, notamment de 20 à 50 % p/p ou de 25 à 45 % p/p.

3. Fibres électrofilées selon la revendication 1, où ledit premier et le second polymères fibrogènes hydrophiles ont une solubilité dans ledit solvant hydrophile qui est égale ou supérieure à 3 g/100 ml à 25 °C ou égale ou supérieure à 10 g/100 ml à 25 °C.

4. Fibres électrofilées selon l'une quelconque des revendications précédentes, où ledit solvant hydrophile est sélectionné parmi l'éthanol ou des mélanges éthanol-eau.

5. Fibres électrofilées selon la revendication 3, où lesdits mélanges éthanol-eau contiennent 20 % v/v d'eau ou moins ou 10 % v/v d'eau ou moins.

6. Fibres électrofilées selon la revendication 3 ou 4, où lesdits mélanges éthanol-eau contiennent 5 % v/v d'eau ou moins, par exemple 3 % v/v d'eau ou moins.

7. Fibres électrofilées selon l'une quelconque des revendications précédentes, où ledit premier polymère hydrophile a une solubilité dans l'eau qui est au moins 50 fois plus élevée que la solubilité dans l'eau dudit second polymère hydrophile, l'une et l'autre mesurées à 37 °C.

8. Fibres électrofilées selon l'une quelconque des revendications précédentes, où ledit premier polymère hydrophile a une solubilité dans l'eau qui est au moins 100 fois plus élevée ou au moins 500 fois plus élevée que la solubilité dans l'eau dudit second polymère hydrophile, l'une et l'autre mesurées à 37 °C.

9. Fibres électrofilées selon l'une quelconque des revendications précédentes, où la substance bioadhésive est au plus très légèrement soluble dans ledit solvant sélectionné parmi l'éthanol et des mélanges éthanol-eau à une température de 25 °C.

10. Fibres électrofilées selon l'une quelconque des revendications précédentes, où la substance bioadhésive a une solubilité de 0,1 % p/p au plus dans ledit solvant sélectionné parmi l'éthanol et des mélanges éthanol-eau à une température de 25 °C.

11. Fibres électrofilées selon l'une quelconque des revendications précédentes, où la substance bioadhésive a une solubilité de 0,01 % p/p au plus dans ledit solvant sélectionné parmi l'éthanol et des mélanges éthanol-eau à une température de 25 °C.

12. Fibres électrofilées selon l'une quelconque des revendications précédentes, où au moins 90 % p/p de la substance bioadhésive est présente sous une forme solide.

13. Fibres électrofilées selon l'une quelconque des revendications précédentes, où ledit premier polymère hydrophile est sélectionné parmi une polyvinylpyrrolidone (PVP), l'hydroxypropylcellulose et des mélanges de celles-ci.

14. Fibres électrofilées selon l'une quelconque des revendications précédentes, où ledit second polymère hydrophile est sélectionné parmi l'éthylcellulose, des acrylates et des copolymères acryliques (Eudragit®) et des mélanges de ceux-ci.

15. Fibres électrofilées selon l'une quelconque des revendications précédentes, où le premier polymère hydrophile est une PVP et le second polymère hydrophile est un copolymère acrylique comme qu'un Eudragit®.

16. Fibres électrofilées selon l'une quelconque des revendications précédentes, où la substance bioadhésive est sélectionnée parmi les suivantes : dextranes, poly(oxydes d'éthylène) (PEO), alginate, tragacanthe, carraghénane, pectine, gélatine, guar, xanthane, gellane, méthylcellulose, hydroxypropylméthylcellulose (HPMC), poly(alcool vinylique) (PVA), polymères d'acides acryliques [dérivés de poly(acides acryliques), PAA], chitosane, lectines, polymères thiolés, Polyox WSR-A, PAA-co-PEG (PEG = polyéthylène glycol) et mélanges de ceux-ci.

**17.** Fibres électrofilées selon l'une quelconque des revendications précédentes, où la substance bioadhésive est un dextrane d'un poids moléculaire moyen qui va de 400 000 Da à 2 000 000 Da.

**18.** Fibres électrofilées selon l'une quelconque des revendications précédentes, où la substance bioadhésive est un dextrane d'un poids moléculaire moyen de 2 000 000 Da environ.

**19.** Fibres électrofilées selon l'une quelconque des revendications 1-16, où la substance bioadhésive est un poly(oxyde d'éthylène) d'un poids moléculaire moyen qui va de 100 000 Da à 4 000 000 Da.

**20.** Fibres électrofilées selon l'une quelconque des revendications 1-16, où la substance bioadhésive est un poly(oxyde d'éthylène) d'un poids moléculaire moyen de 2 000 000 Da.

**21.** Fibres électrofilées selon l'une quelconque des revendications précédentes, où le rapport en poids entre la substance bioadhésive et les polymères fibrogènes hydrophiles dans les fibres est dans une plage qui va de 0,1 à 10.

**22.** Fibres électrofilées selon l'une quelconque des revendications précédentes, où la substance médicamenteuse est sélectionnée parmi des substances médicamenteuses qui sont indiquées dans le traitement d'une maladie de la peau ou des muqueuses.

**23.** Fibres électrofilées selon l'une quelconque des revendications précédentes, où la substance médicamenteuse est sélectionnée parmi des substances médicamenteuses qui sont indiquées dans le traitement d'une maladie de la cavité buccale ou vaginale.

**24.** Fibres électrofilées selon la revendication 23, où la substance médicamenteuse est sélectionnée parmi des substances médicamenteuses qui sont indiquées dans le traitement local d'une maladie de la cavité buccale.

**25.** Fibres électrofilées selon l'une quelconque des revendications précédentes, où la teneur en eau est au plus de 5 % p/p environ.

**26.** Fibres électrofilées selon l'une quelconque des revendications précédentes pour une utilisation en médicine.

**27.** Fibres électrofilées selon l'une quelconque des revendications précédentes pour une utilisation dans le traitement de maladies de la cavité buccale ou vaginale.

**28.** Composition comprenant des fibres électrofilées telles que définies à l'une quelconque des revendications 1-27.

**29.** Composition selon la revendication 28, où la concentration en fibres électrofilées dans la composition va de 70 à 100 % p/p.

**30.** Composition selon la revendication 28 ou 29 sous la forme d'une composition en couches.

**31.** Fibres électrofilées selon l'une quelconque des revendications 1-27 ou composition selon l'une quelconque des revendications 28-30, où une surface extérieure desdites fibres électrofilées ou de ladite composition est fournie dotée d'un revêtement.

**32.** Fibres électrofilées ou composition selon la revendication 31, où le revêtement est imperméable à l'eau.

**33.** Fibres électrofilées ou composition selon la revendication 31 ou 32, où le revêtement comprend un carbothane, une polycaprolactone ou un poly(éthylène-co-acétate de vinyle) ou des mélanges de ceux-ci.

**34.** Fibres électrofilées telles que définies à l'une quelconque des revendications 1-27 ou composition telle que définie à l'une quelconque des revendications 28-33 pour une utilisation en médecine.

**35.** Fibres électrofilées selon l'une quelconque des revendications 1-27 ou composition telle que définie à l'une quelconque des revendications 28-35 pour une utilisation dans le traitement de maladies de la cavité buccale.

**36.** Nécessaire comprenant :

i) des fibres électrofilées ou une composition telles que définies à l'une quelconque des revendications précédentes et
ii) un applicateur pour appliquer la composition dans la cavité buccale.

**37.** Procédé de préparation de fibres électrofilées telles que définies à l'une quelconque des revendications 1-27, le procédé comprenant

i) la dissolution du premier et du second polymères hydrophiles dans un solvant sélectionné parmi l'éthanol ou des mélanges éthanol-eau,
ii) la mise en suspension de la substance bioadhésive dans la solution résultant de l'étape i),
iii) le cas échéant, l'addition de la substance médicamenteuse à la dispersion résultant de l'étape ii),
iv) l'électrofilage du mélange résultant de l'étape ii) ou, le cas échéant, de l'étape iii),

où lesdits premier et second polymères hydrophiles sont solubles dans ledit solvant, et où ladite substance bioadhésive est au plus légèrement soluble dans ledit solvant,
pour obtenir des fibres électrofilées, où au moins 90 % p/p de la substance bioadhésive est présente sous une forme solide.

**38.** Procédé de préparation de fibres électrofilées selon l'une quelconque des revendications 1-27, le procédé comprenant

i) la dissolution du premier et du second polymères hydrophiles dans un solvant sélectionné parmi l'éthanol, l'eau ou des mélanges de ceux-ci pour obtenir une première solution,
ii) le cas échéant, la dissolution ou la mise en suspension de la substance médicamenteuse dans ladite solution pour obtenir un mélange,
iii) la mise en suspension de la substance bioadhésive dans le solvant pour obtenir une dispersion,
iv) l'électrofilage à deux filières du mélange obtenu à l'étape i) ou, le cas échéant, à l'étape ii) et de la dispersion obtenue à l'étape iii),

où lesdits premier et second polymères hydrophiles sont solubles dans ledit solvant, et où ladite substance bioadhésive est légèrement soluble dans ledit solvant,
pour obtenir des fibres électrofilées, où au moins 90 % p/p de la substance bioadhésive est présente sous une forme solide.

**39.** Procédé selon la revendication 37 ou 38 comprenant en outre une étape de revêtement d'une surface extérieure des fibres avec un polymère hydrophobe.

**40.** Procédé selon la revendication 39, où les fibres revêtues sont soumises à un chauffage pour faire fondre ou ramollir le polymère hydrophobe.

Figure 1A

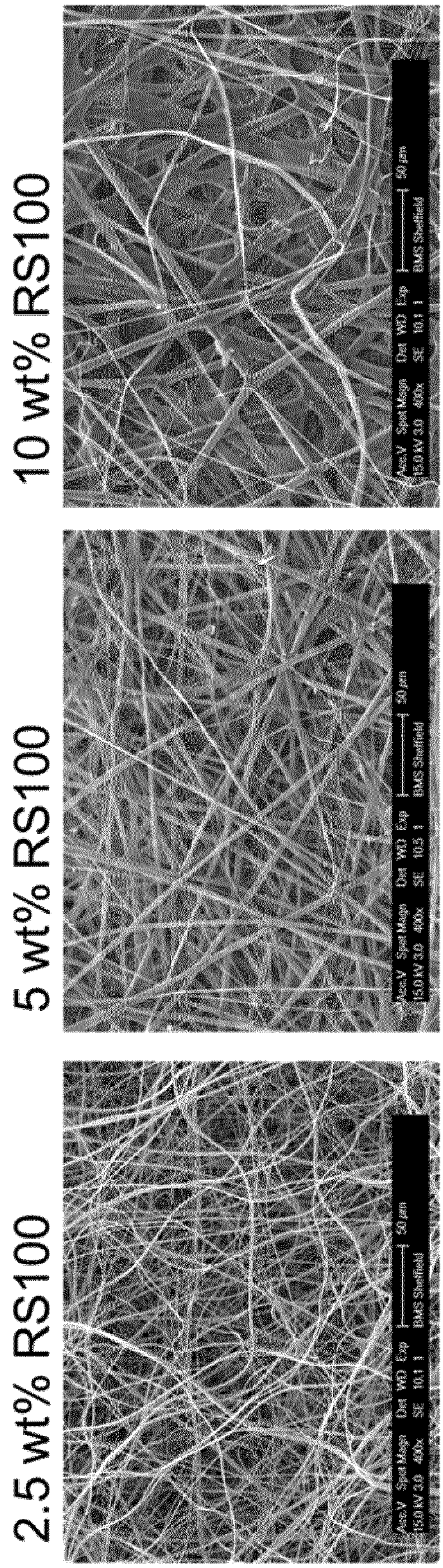

Figure 1B

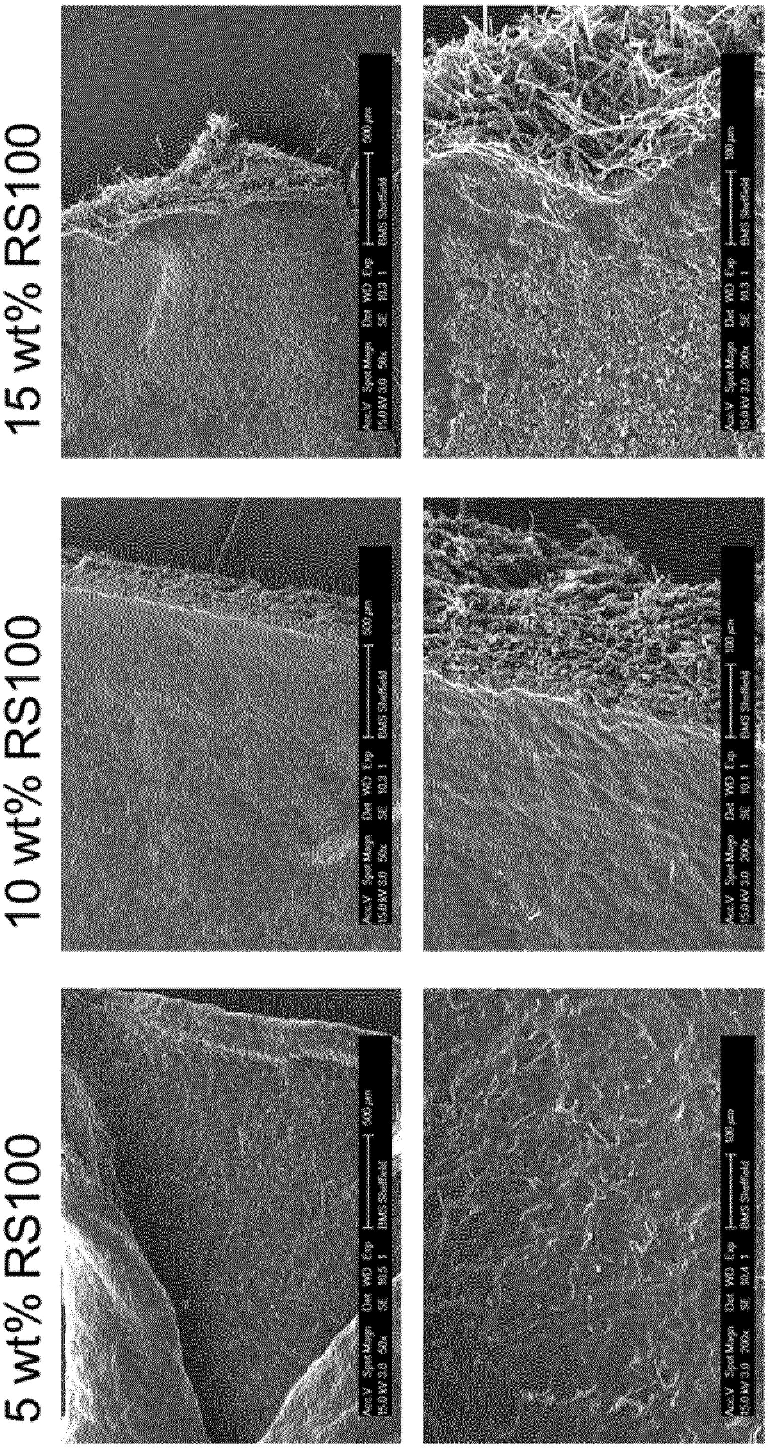

Figure 2

PVP + 10 wt% Dextrans | PVP + 10 wt% PEO

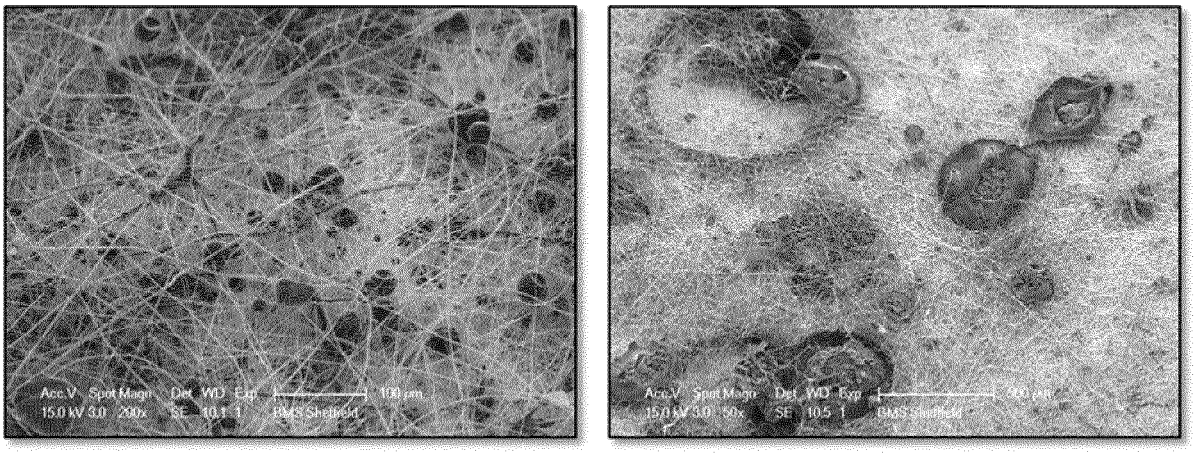

Figure 3

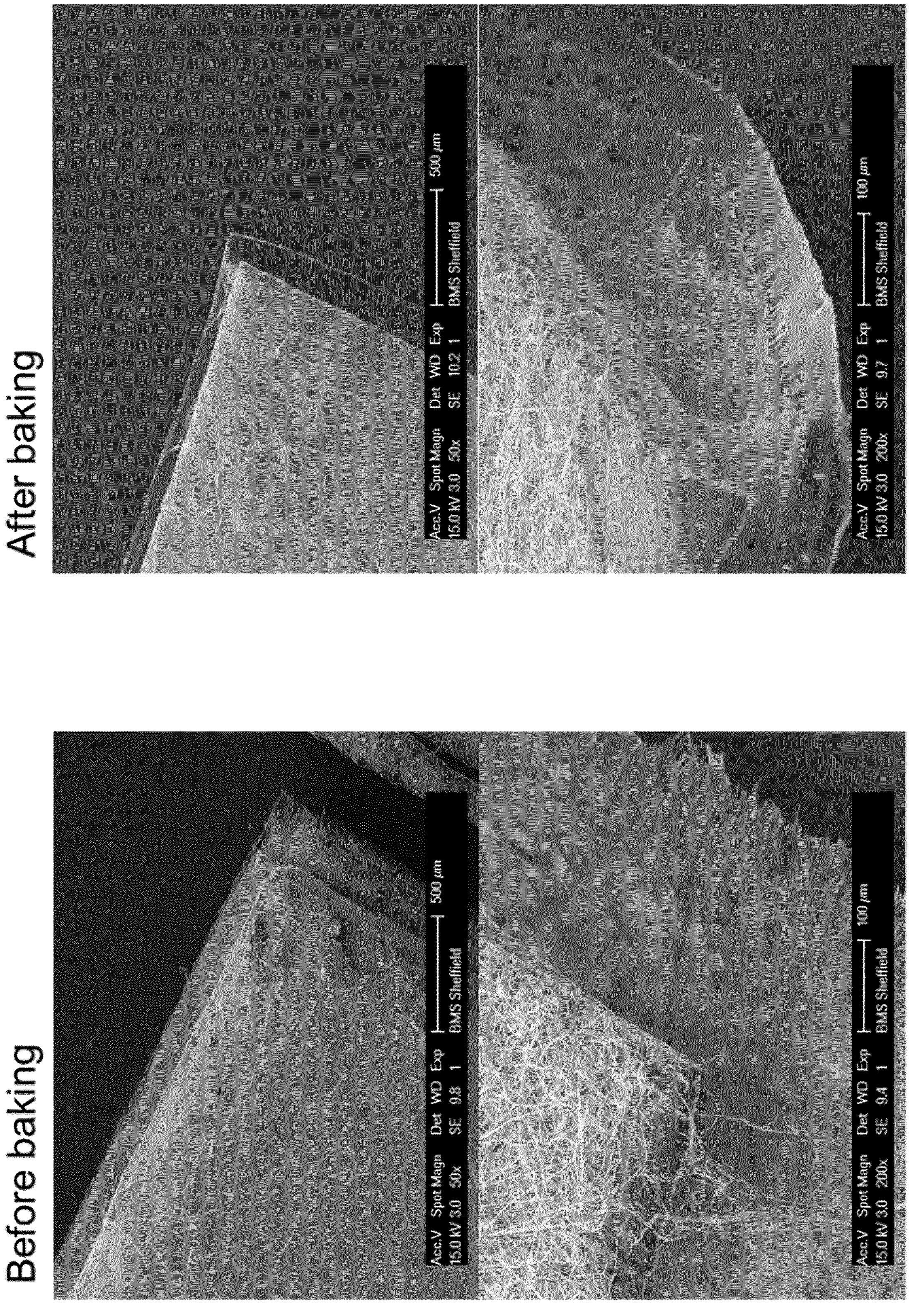
After baking
Before baking
Acc.V Spot Magn Det WD Exp
15.0 kV 3.0 50x SE 10.2 1
500 µm
BMS Sheffield
Acc.V Spot Magn Det WD Exp
15.0 kV 3.0 200x SE 9.7 1
100 µm
BMS Sheffield
Acc.V Spot Magn Det WD Exp
15.0 kV 3.0 50x SE 9.8 1
500 µm
BMS Sheffield
Acc.V Spot Magn Det WD Exp
15.0 kV 3.0 200x SE 9.4 1
100 µm
BMS Sheffield

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4765983 A
- WO 2004014304 A2
- WO 0127365 A1
- EP 2810645 A1
- WO 2014066297 A1
- WO 2015189212 A1
- EP 2015062842 W
- WO 2015189212 A